(19) Europäisches Patentamt
European Patent Office
Office européen des brevets

(11) EP 2 314 572 A1

(12) EUROPÄISCHE PATENTANMELDUNG

(43) Veröffentlichungstag:
**27.04.2011 Patentblatt 2011/17**

(21) Anmeldenummer: **09173567.0**

(22) Anmeldetag: **20.10.2009**

(51) Int Cl.:
***C07C 303/34*** *(2006.01)* ***C07C 303/40*** *(2006.01)*
***C07C 307/02*** *(2006.01)* ***C07C 311/08*** *(2006.01)*
***C07C 311/09*** *(2006.01)* ***C07C 311/10*** *(2006.01)*
***C07C 311/21*** *(2006.01)* ***C07F 1/02*** *(2006.01)*
***H01M 10/0525*** *(2010.01)* ***H01M 10/0568*** *(2010.01)*

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(71) Anmelder: **PHILIPPS-UNIVERSITÄT MARBURG
35037 Marburg (DE)**

(72) Erfinder:
- **Sundermeyer, Jörg, Prof. Dr.
35041 Marburg (DE)**
- **Roling, Bernhard, Prof. Dr.
35039 Marburg (DE)**
- **Linder, Thomas, Dr.
Calgary AB
Alberta T2L 1Z9 (CA)**
- **Frömling, Till
1120 Wien (AT)**
- **Huber, Benedikt
66606 Sankt Wendel (DE)**

(74) Vertreter: **Stumpf, Peter
Kerkrader Straße 3
35394 Gießen (DE)**

(54) **Lithiumsalze von Pentafluorphenylamid-Anionen, ihre Herstellung und ihre Verwendung**

(57) Die vorliegende Erfindung stellt neue, Pentafluorphenylamidanionen umfassende Lithiumsalze der allgemeinen Formel $Li^+[N(SO_2-R)(C_6F_5)]^-$ bereit, die optional als lösungsmittelfreie Komplexe vorliegen. Dabei ist R ausgewählt aus Fluor, linearen oder verzweigten acyclischen oder cyclischen Alkylgruppen mit 1 bis 20 Kohlenstoffatomen, die nicht fluoriert, partiell fluoriert oder vollständig fluoriert sind; oder nicht fluorierten, partiell fluorierten oder vollständig fluorierten Aryl- oder Benzylgruppen mit bis zu 20 Kohlenstoffatomen. Die erfindungsgemäßen Lithiumsalze werden hergestellt, indem die korrespondierende NH-Säure des Pentafluorphenylamids mit einem Äquivalent Lithium-bis-trimethylsilyamid oder Lithiumorganyl umgesetzt wird, wobei die Reaktion vorteilhaft in Gegenwart von apolar aprotischen Lösungsmitteln durchgeführt wird. Auf diese Weise werden Lithiumsalze in Form von lösungsmittelfreien Komplexen erhalten. Diese lösungsmittelfreien Lithiumkomplexe sind thermisch, elektrochemisch und gegen Oxidationen stabil und weisen eine hohe Ionenleitfähigkeit auf. Die erfindungsgemäßen Lithiumsalze können als Ionen leitende Materialien, elektrisch leitende Materialien, Farbstoffe und in der chemischen Katalyse verwendet werden. Bevorzugt werden sie als Ionen leitende Elektrolyte in Lithium-Ionenakkus verwendet.

EP 2 314 572 A1

## Beschreibung

Die vorliegende Erfindung stellt neue, Pentafluorphenylamidanionen umfassende Lithiumsalze sowie ein Verfahren zu ihrer Herstellung bereit. Erfindungsgemäß werden diese Lithiumsalze hergestellt, indem die korrespondierende NH-Säure des Pentafluorphenylamids mit einer äquimolaren Menge Lithium-bis-trimethylsilylamid oder Lithiumorganyl umgesetzt wird. Die Reaktion wird in Gegenwart von unpolar aprotischen oder dipolar aprotischen Lösungsmitteln durchgeführt. Bei Verwendung unpolar aprotischer Lösungsmittel werden Lithiumsalze erhalten, die als lösungsmittelfreie Komplexe vorliegen. Durch die Abwesenheit von Lösungsmitteln wie beispielsweise Ether sind die erfindungsgemäßen Pentafluorphenylamidanionen umfassenden Lithiumsalze thermisch und gegen Oxidation stabiler als die entsprechenden lösungsmittelhaltigen Komplexe und können als Ionen leitende Materialien, elektrisch leitende Materialien, Farbstoffe und in der chemischen Katalyse verwendet werden. Bevorzugt werden sie als Ionen leitende Elektrolyte in Lithium-Ionenakkus verwendet.

## Beschreibung und Einleitung des allgemeinen Gebietes der Erfindung

Die vorliegende Erfindung betrifft die Gebiete metallorganische Chemie und Elektrochemie.

## Stand der Technik

Im Hinblick auf die rasch fortschreitende Entwicklung von auf Lithiumionen basierenden Akkumulatoren besteht ein wachsender Bedarf an Lithiumsalzen, die als Ionen leitende Elektrolyte in diesen Akkumulatoren eingesetzt werden können.

Die idealen Kandidaten sollten in nichtwässrigen dipolaren aprotischen Lösungsmitteln oder ionischen Flüssigkeiten vollständig löslich sein. Werden diese Lithiumsalze in ionischen Flüssigkeiten gelöst, so sollten sie darin in mono- oder oligonukleare Ionen mit hoher Ionenbeweglichkeit dissoziieren. Vor allem hohe Überführungszahlen des Lithiumions sind erforderlich.

Die Anforderungen im Hinblick auf das Anion sind chemische und elektrochemische Stabilität, besonders gegenüber oxidativem Abbau, sowie Inertheit gegenüber Lithium in Temperaturbereichen zwischen 30 und 120°C, außerdem thermische Stabilität und eine geringe Toxizität.

Bislang gibt es kein Lithiumsalz, das alle diese Anforderungen in vollem Umfang erfüllt. Unter den bisher verwendeten Salzen sind $LiClO_4$, $LiBF_4$ und $LiPF_6$ die am häufigsten eingesetzten. Außerdem gilt $LiN(SO_2CF_3)$ als viel versprechend.

Des Weiteren kennt der Fachmann Lithium-bis-pentafluorphenylamid (Li-BPFPA). Bis(pentafluorphenylamin) (BPFPA) ist beispielsweise durch Reaktion von $LiNH_2$ mit $C_6F_6$ erhältlich. Dies ist in R Koppang: "Use of a Lithium Amide Suspension in Tetrahydrofuran for Preparation of Some Polyfluorophenyl- and Polyfluorodiphenylamines", Acta Chem Scand 1971, 25, 3067-3071 beschrieben. Das Lithiumsalz von BPFPA ist durch Reaktion von BPFPA mit n-Butyllithium in Hexan erhältlich. Li-BPFPA löst sich nur wenig in Kohlenwasserstoffen, ist aber gut löslich in dipolar aprotischen Lösungsmitteln oder Ether. Dies ist in A Khvorost, PL Shutov, K Harms, J Lorberth, J Sundermeyer, SS Karlov, GS Zaitseva: "Lithium Bis(pentafluorophenyl)amides - Synthesis and Characterization of its Complexes with Diethyl Ether and THF", Z. Anorg. Allg. Chem. 2004, 630, 885-889 beschrieben.. Etherate sind jedoch für thermische und insbesondere für elektrochemische Anwendungen nicht geeignet.

Die WO 2009/003224 A1 beschreibt Lithiumenergiespeichervorrichtungen, die einen ionischen flüssigen Elektrolyten umfassend Bis-fluorsulfonylimid als Anion und ein Kation als Gegenion sowie Lithiumionen enthalten. Pentafluorphenylamidanionen umfassende Lithiumsalze werden nicht offenbart.

Die US 6,319,428 B1 beschreibt Ionen leitende Substanzen mit Anionen der allgemeinen Formel $[R_f\text{-}SO_x\text{-}N\text{-}Z]^-$. Rf ist dabei eine perfluorierte Gruppe, aber niemals eine Pentafluorphenylgruppe; Z ist eine Elektronen ziehende Gruppe, $SO_x$ steht für eine Sulfonyl- oder Sulfinylgruppe, und als Gegenionen werden einwertige Metallkationen verwendet. Lithiumsalze der beschriebenen Anionen werden durch Ionenaustausch aus dem korrespondierenden Kaliumsalz hergestellt, indem Lithiumchlorid in THF zugegeben wird.

Die US 2007/0093678 A1 beschreibt Perfluoralkylsulfonamidverbindungen der allgemeinen Formel $Y^+[N(SO_2R^f)(CF_3)]^-$. Darin ist $Y^+$ ein beliebiges organisches oder anorganisches Kation, und $R^f$ ist eine perfluorierte Alkylgruppe mit 1 bis 4 Kohlenstoffatomen. In keinem Fall umfasst das Anion perfluorierte Arylgruppen.

In T Linder, J Sundermeyer: "Three novel anions based on pentafluorophenyl amine combined with two new synthetic strategies for the synthesis of highly lipophilic ionic liquids", Chem Commun 2009, 2914-2916 werden ionische Flüssigkeiten mit Bis-Pentafluorphenylamid, Pentafluorphenyltrifluormethylsulfonylimid und Pentafluorphenylnonafluorbutylsulfonylimid als Anionen und Imidazolium- und Phosphoniumionen als Kationen beschrieben. Der Artikel liefert jedoch keinen Hinweis auf die Herstellung etherfreier Lithium-Pentafluorphenylimidverbindungen oder deren thermische und elektrochemische Eigenschaften.

Die WO 95/26056 beschreibt Ionen leitende Materialien, die wenigstens eine ionische Verbindung in einem aprotischen Solvens umfasst, wobei die ionische Verbindung eine Verbindung der Formel $(1/mM)^+[(ZY_2)N]^-$. Darin ist M ein Metall, m dessen Wertigkeit, Y ist $SO_2$ oder POZ, und jeder Substituent Z ist unabhängig voneinander ein Fluoratom, eine optional perfluorierte organische Gruppe die optional wenigstens eine polymerisierbare Gruppe aufweist, wobei mindestens einer der Substituenten Z ein Fluoratom darstellt. Diese Patentanmeldung offenbart jedoch kein Verbindungen, bei denen eine Pentafluorphenylgruppe direkt an ein Stickstoffatom gebunden ist.

Die WO 2007 / 131498 A2 beschreibt hydrophobe ionische Flüssigkeiten aus Pentafluorphenylimidanionen und organischen oder anorganischen Kationen, wobei anorganische Kationen aus Alkali- oder Erdalkalimetallkationen ausgewählt sind. Es findet sich jedoch kein Hinweis auf die besondere Eignung von Lithium-Pentafluorphenylamidionen als Ionen leitende Elektrolyte in Lithium-Ionenakkus, und die dort beschriebenen Herstellungsverfahren verwenden unter Anderem Ether.

Die vorliegende Erfindung überwindet die Nachteile des Standes der Technik, indem sie eine neue Klasse von Lithiumsalzen mit schwach koordinierenden Anionen bereitstellt. Diese neuen Lithiumsalze leiten sich formal von den Lithiumsalzen der Di-Perfluoralkylsulfonylamide und Di-Fluorsulfonylamide ab, indem eine Perfluoralkylsulfonyl- bzw. Fluorsulfonyl-gruppe durch eine elektronenziehende Pentafluorphenylgruppe (Pfp) ersetzt wird. Außerdem stellt die vorliegende Erfindung ein neues Verfahren bereit, um diese Lithiumsalze frei von koordinierenden Ethermolekülen herzustellen. Die erfindungsgemäßen neuen Lithiumsalze sind thermisch stabiler und weisen eine höhere Ionenbeweglichkeit auf als bekannte Lithiumsalze.

## Aufgabe

Aufgabe der Erfindung ist es, neue Lithiumsalze mit verbesserter thermischer Stabilität und verbesserter Ionenbeweglichkeit sowie Verfahren zu ihrer Herstellung bereitzustellen.

## Lösung der Aufgabe

Die Aufgabe der Bereitstellung neuer Lithiumsalze mit verbesserter thermischer Stabilität und verbesserter Ionenbeweglichkeit bereitzustellen wird erfindungsgemäß gelöst durch Pentafluorphenylamidoanionen umfassende Lithiumsalze gemäß Formel (1)

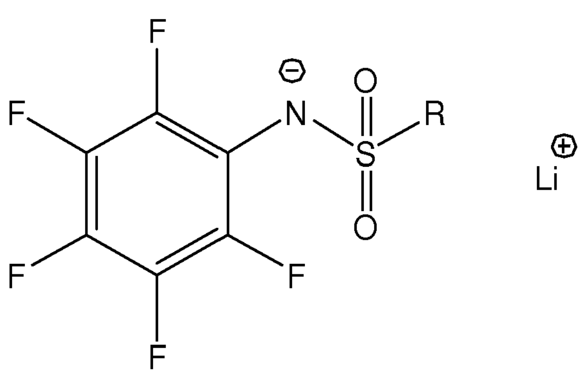

(I)

,

worin R ausgewählt ist aus

- F (Fluorid),
- linearen oder verzweigten acyclischen oder cyclischen Alkylgruppen mit 1 bis 20 Kohlenstoffatomen, die nicht fluoriert, partiell fluoriert oder vollständig fluoriert sind,
- nicht fluorierte, partiell fluorierte oder vollständig fluorierte Aryl- oder Benzylgruppen mit bis zu 20 Kohlenstoffatomen.

Überraschend wurde gefunden, dass die erfindungsgemäßen, Pentafluorphenylamidoanionen umfassenden Lithiumsalze eine höhere thermische Stabilität und eine höhere Ionenbeweglichkeit aufweisen als bisher bekannte Lithiumsalze. Die erfindungsgemäßen Verbindungen gemäß Formel (1) werden nachfolgend auch als Lithium-Pentafluorphenylamidsalze bezeichnet.

Die erfindungsgemäßen Lithium-Pentafluorphenylamidsalze sowie das Verfahren zu ihrer Herstellung sind nachfolgend erläutert, wobei die Erfindung alle nachfolgend aufgeführten Ausführungsformen einzeln und in Kombination

miteinander umfasst.

In einer bevorzugten Ausführungsform ist R ein Fluoratom.

In einer weiteren bevorzugten Ausführungsform ist R eine nicht fluorierte, partiell fluorierte oder vollständig fluorierte Alkylgruppe mit 1 bis 20 Kohlenstoffatomen, wobei die Alkylgruppe linear oder verzweigt, cyclisch oder acyclisch sein kann. Besonders bevorzugt handelt es sich um eine vollständig fluorierte, d.h. eine um eine perfluorierte Alkylgruppe.

Im Rahmen der vorliegenden Erfindung werden lineare und verzweigte acyclische Alkylgruppen mit 1 bis 20 Kohlenstoffatomen ausgewählt aus Methyl, Ethyl, n-Propyl, Isopropyl, 1-Butyl, 2-Butyl, tert.-Butyl, 1-Pentyl, 2-Pentyl, 3-Pentyl, 3-Methylbutyl, 2,2-Dimethylpropyl, sowie allen Isomere von Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl.

Dem Fachmann ist bekannt, dass cyclische Alkylgruppen mindestens drei Kohlenstoffatome enthalten müssen. Dementsprechend umfassen cyclische Alkylgruppen im Rahmen der vorliegenden Erfindung Propyl-, Butyl-, Pentyl-, Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl-, Octadecyl-, Nonadecyl-, Eicosyl-Ringe mit 3 bis 20 Kohlenstoffatomen. Eine cyclische Alkylgruppe im Sinne der vorliegenden Erfindung wird ausgewählt aus den genannten ringförmigen Alkylgruppen, die keine weiteren Substituenten tragen, und aus den genannten ringförmigen Alkylgruppen, welche ihrerseits an eine oder mehrere acyclische Alkylgruppen gebunden sind. Im letztgenannten Fall kann die Bindung der cyclischen Alkylgruppe an die S02-Gruppe gemäß Formel I über ein cyclisches oder ein acyclisches Kohlenstoffatom der cyclischen Alkylgruppe erfolgen. Cyclische Alkylgruppen enthalten gemäß obiger Definition des Begriffes "Alkylgruppe" ebenfalls insgesamt maximal 20 Kohlenstoffatome.

In einer weiteren bevorzugten Ausführungsform ist R eine nicht fluorierte, partiell fluorierte oder vollständig fluorierte Aryl- oder Benzylgruppe mit bis zu 20 Kohlenstoffatomen. Arylgruppen werden dabei ausgewählt aus Phenyl, Naphthyl, Anthracenyl, Phenanthrenyl, Tetracenyl, Biphenyl, Terphenyl. Besonders bevorzugt handelt es sich um eine vollständig fluorierte, d.h. eine um eine perfluorierte Benzyl- oder Arylgruppe.

Optional kann die Aryl- oder Benzylgruppe mit einer bis drei linearen oder verzweigten Alkylgruppen substituiert sein, wobei diese Alkylgruppen nicht fluoriert, partiell fluoriert oder vollständig fluoriert sein können. Dabei enthalten auch mit ein bis drei Alkylgruppen substituierte Aryl- oder Benzylgruppen bis zu 20 Kohlenstoffatome.

Ganz besonders bevorzugt wird R ausgewählt aus F, Trifluormethyl und Nonafluor-n-butyl.

In besonders vorteilhaften Ausführungsformen von Verbindungen gemäß Formel I ist kein Lösungsmittelmolekül an der Koordination des Lithiumatoms beteiligt, insbesondere keine Ethermoleküle.

Die erfindungsgemäßen Lithium-Pentafluorphenylamidsalze gemäß Formel (I) werden hergestellt, indem die freie Säure des korrespondierenden Pentafluorphenylamids gemäß Formel (II) mit äquimolaren Mengen von Lithiumbistrimethylsilylamid oder einem Lithiumorganyl umgesetzt wird. Dies ist exemplarisch für die Reaktion mit Lithiumbistrimethylsilylamid gezeigt:

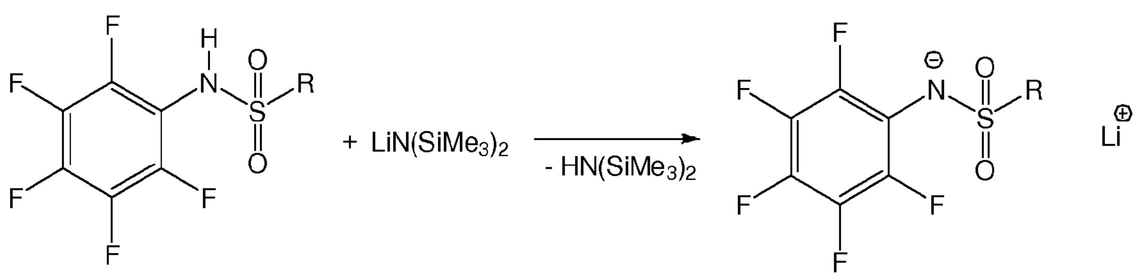


(II) (I)

Die Reaktion wird bei einer Temperatur von etwa 20 bis 80 ˚C über 12 Stunden unter Rühren in einem unpolar aprotischen oder dipolar aprotischen Lösungsmittel durchgeführt. Die freie Säure des Lithium-Pentafluorphenylamids wird dabei unter Erwärmen auf 30 bis 60˚C im Lösungsmittel gelöst. Anschließend wird Lithium-bistrimethylsilylamid oder ein Lithiumorganyl wie tert.-Butyllithium zugegeben. Besonders vorteilhaft wird mit Lithiumbistrimethylsilylamid gearbeitet, das zuvor in demselben Lösungsmittel gelöst wird wie die freie Säure des Lithiumpentafluorphenylamids. Optional kann Lithiumbistrimethylsilylamid oder das Lithiumorganyl auch als Feststoff zur Lösung der freien Säure des Lithiumpentafluorphenylamids gegeben werden.

Unter "Lithiumorganyl" werden dabei alle Verbindungen verstanden, die eine Lithium-Kohlenstoff-Bindung enthalten und bei denen es sich nicht um Lithiumcyanid handelt.

Beide Reaktanden müssen nicht zwingend im selben Lösungsmittel gelöst werden. Die Reihenfolge der Zugabe kann auch umgekehrt erfolgen, d.h. das Lithiumbistrimethylsilylamid oder Lithiumorganyl wird vorgelegt, und die NH-Säure wird zugegeben. Die Gesamtkonzentration der beiden Reaktanden in oben genannten Lösungsmitteln beträgt dabei vorteilhaft 0,01 mol/L bis 0,5 mol/L.

Es ist empfehlenswert, die beiden Reaktanden zunächst zu lösen und danach wie oben beschrieben miteinander zu vermischen. In diesem Fall ist es vorteilhaft, etwa die Hälfte bis zwei Drittel des insgesamt einzusetzenden Lösungsmittels zum Lösen der freien Säure des Pentafluorphenylamids zu verwenden und mit dem restlichen Lösungsmittel das Lithiumtrimethylsilylamid oder das Lithiumorganyl zu lösen.

Nach 12 h bei 80 ˚C wird das Reaktionsgemisch auf Raumtemperatur abgekühlt, dann werden die flüchtigen Bestandteile bei entfernt, der Rückstand mit einem unpolar aprotischen oder dipolar aprotischen Lösungsmittel gewaschen getrocknet. Das Entfernen der flüchtigen Bestandteile sowie das Trocknen erfolgen vorteilhaft bei vermindertem Druck.

Das unpolar aprotische oder dipolar aprotische Lösungsmittel wird ausgewählt aus aliphatischen, ungesättigten und aromatischen Kohlenwasserstoffen wie beispielsweise Toluol, partiell oder voll halogenierten Kohlenwasserstoffen (z.B. Chlorbenzol, Chloroform, Tetrachlorkohlenstoff, FCKW, FKW, Frigene) sowie Petrolether. Bevorzugtes Lösungsmittel ist Toluol. Dipolar aprotische Lösungsmittel, in denen die Synthese ebenfalls erfolgen kann, sind beispielsweise Diethylether, Tetrahydrofuran und andere cyclische und acyclische Ether, weiterhin geeignet sind DMF, DMSO, cyclische und acyclische Diorganocarbonate, Carbonsäureester und Carbonsäure-diorganoamide, Pyridin und tertiäre Amine. In diplolar aprotischen Lösungsmitteln werden in der Regel Komplexe des Lithiumkations mit den Donorfunktionen dieser Lösungsmittel isoliert, beispielsweise Lithiumetherate. Daher ist die Synthese in apolar aprotischen Lösungsmitteln der Synthese in dipolar aprotischen Lösungsmitteln vorzuziehen.

Dem Fachmann ist jedoch bekannt, dass Lösungsmittelliganden - beispielsweise Etherliganden - am Lithiumatom durch andere Liganden, wie beispielsweise ionische Flüssigkeiten oder Organocarbonate, verdrängt werden können. Durch diese Verdrängungsreaktion lassen sich lösungsmittelfreie erfindungsgemäße Lithiumsalze gemäß Formel I aus den korrespondierenden, mit Lösungsmitteln koordinierten Komplexen herstellen.

"Verminderter Druck" ist ein Druck unterhalb des Normdrucks von 1.013 mbar. Vorteilhaft werden die flüchtigen Bestandteile mit Hilfe eines Vakuums von 0,000001 mbar bis 10 mbar entfernt.

Zum Waschen des Rückstandes eignen sich die oben aufgeführten unpolar aprotischen, sauerstofffreien Lösungsmittel. Besonders geeignet sind beispielsweise n-Pentan, Cyclopentan, n-Hexan, Cyclohexan und n-Heptan.

Das erfindungsgemäße Verfahren umfasst damit folgende Schritte:

a) Lösen der freien Säure des Pentafluorphenylamids gemäß Formel II

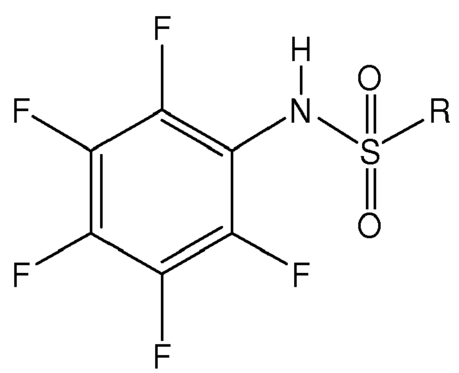

(II)

in einem unpolar aprotischen oder dipolar aprotischen Lösungsmittel unter Erwärmen,

b) Mischen mit einer äquimolaren Menge von Lithiumbistrimethylsilylamid oder eines Lithiumorganyls als Feststoff oder in Lösung,

c) Einstellen des so erhaltenen Gemisches aus freier Säure des Pentafluorphenylamids und des Lithiumtrimethylsilylamids oder des Lithiumorganyls auf eine Gesamtkonzentration dieser beiden Reaktanden von 0,01 mol/L bis 0,5 mol/L,

d) Rühren bei 80 ˚C für 12 Stunden,

e) Abkühlen des Reaktionsgemisches auf Raumtemperatur,

f) Entfernen der flüchtigen Bestandteile des Gemisches,

g) Waschen des Rückstandes mit einem unpolar aprotischen oder dipolar aprotischen Lösungsmittel und anschließendes Trocknen.

In einer bevorzugten Ausführungsform wird das Lithiumbistrimethylsilylamid oder das Lithiumorganyl gemäß Schritt b) in Lösung zugegeben.

In einer weiteren Ausführungsform wird das Lithiumbistrimethylsilylamid oder das Lithiumorganyl gemäß Schritt b) als Feststoff zugegeben.

In einer weiteren Ausführungsform erfolgt das Mischen gemäß Schritt b) indem eine Lösung des Lithiumbistrimethylsilylamids oder des Lithiumorganyls vorgelegt und dann die Lösung des Pentafluorphenylamids gemäß Schritt a) zugegeben wird.

Ganz besonders bevorzugt wird das erfindungsgemäße Verfahren unter Verwendung unpolar aprotischer Lösungsmittel durchgeführt. Dies bezieht sich gleichermaßen auf das Lösen der freien Säure des Pentafluorphenylamids gemäß Formel II (in Schritt a) als auch auf das optionale Lösen des Lithiumbistrimethylsilylamids oder des Lithiumorganyls in Schritt b) und auf das Waschen des Rückstandes gemäß Schritt g).

Das erfindungsgemäße Verfahren ist vorteilhaft, da es die erfindunsgemäßen Lithium-Pentafluorphenylamidsalze in hohen Ausbeuten liefert und pro Mol des gewünschten Produktes nur ein Mol Lithium-bistrimethylsilylamid oder Lithiumorganyl eingesetzt werden muss. $LiN(SiMe_3)_2$ und Lithiumorganyle sind weniger nucleophile Basen als die korrespondierende NH-Säure des Pentafluorphenylamids.

In den Ausführungsbeispielen 1 bis 13 sind Herstellung sowie strukturelle, thermische und elektrische Eigenschaften zweier erfindungsgemäßer Lithiumsalze beschrieben. Bei diesen Lithiumsalzen handelt es sich um Lithium-pentafluorphenyltrifluormethylsulfonylimid (Li-PFTFSI, **3**) und Lithium-pentafluorphenylnonafluorbutylsulfonylimid (Li-PFNFSI, **4**). Herstellung und Eigenschaften dieser beiden erfindungsgemäßen Lithiumsalze werden mit Lithium-bis-pentafluorphenylamid (LiBPFPA, 1), dem daraus durch Erhitzen erhältlichen 5,1 0-bis(Pentafluorphenyl)-5,10-dihydro-octafluorphenazin (**2**) und Lithium-bis(trifluormethylsulfonimid) (LiTFSI, $LiN(SO_2CF_3)_2$) verglichen. Die Substanzen **1, 2** und Li-TFSI sind dem Stand der Technik bekannt; Li-TFSI ist ein dem Fachmann bekannter Standard-Elektrolyt.

Die thermische Stabilität von (1) ist in Ausführungsbeispiel 11 beschrieben. LiBPFPA (**1**) ist im Vergleich zum Standard-Elektrolyten Li-TFSI, der sich bei 360 °C zersetzt, nur begrenzt thermisch stabil. Dies wird in Ausführungsbeispiel 2 gezeigt Daher wurden im Rahmen der vorliegenden Erfindung neue Lithiumsalze hergestellt, ausgehend von den asymmetrisch substituierten Sulfonamiden $(F_5C_6)N(H)SO_2CF_3$ (H-PFTFSI) und $(F_5C_6)N(H)SO_2C_4F_9$ (H-PFNFSI). Diese beiden Verbindungen sind starke NH-Säuren, vergleichbar mit der korrespondierenden Säure $HN(SO_2CF_3)_2$, und bilden eine Reihe ionischer Flüssigkeiten oder sogar kristalliner Hydroxoniumsalze, wenn sie aus wassergesättigtem Ether auskristallisiert werden.

Die erfindungsgemäßen Lithiumsalze und auch die an sich bekannte Substanz LiBPFPA (1) wurden im Rahmen der vorliegenden Erfindung erstmals durch Reaktion von Lithium-bis-trimethylsilylamid $LiN(SiMe_3)_2$ mit der jeweiligen korrespondierenden NH-Säure hergestellt. Dieses neue Verfahren gestattet erstmals die etherfreie Herstellung von Pentafluorphenylamidoanionen umfassenden Lithiumsalzen. Die erfindungsgemäßen Lithiumsalze, welche erstmals nicht als Etherate vorliegen, sind auf Grund ihrer verbesserten thermischen und elektrochemischen Eigenschaften besser als die korrespondierenden Etherate für die Verwendung als elektrisch leitende Elektrolyte geeignet.

Die erfindungsgemäßen Verbindungen Li(PFTFSI) **3** und Li(PFNFSI) **4** wurden in Form farbloser Pulver erhalten, die in Toluol und anderen Kohlenwasserstoffen unlöslich sind. Sie sind jedoch gut löslich in dipolar aprotischen Lösungsmitteln wie Diethylether, THF, Dimethylcarbonat, DMF und DMSO.

Die Ergebnisse der impedanzspektroskopischen Messungen (siehe Ausführungsbeispiel 13) zeigen eine Korrelation zwischen der Aktivierungsentropie $S_A^{act}$ und der Symmetrie der Anionen. Das TFSI-Anion ist das symmetrischste dieser Anionen, was zu einer hohen Packungsdichte der Anionen im Kristall führt. Eine hohe Packungsdichte ist vorteilhaft für die Koordination der Lithiumionen durch Sauerstoff und damit für die Existenz eindeutig definierter Lithium-Gitterplätze mit einer niedrigen potentiellen Energie. Im thermischen Gleichgewicht ist die Konfigurationsentropie der Lithiumionen auf diesen Gitterplätzen niedrig. Durch thermische Aktivierung werden Lithiumionen auf Zwischengitterplätzen (interstitiell) eingebaut, und es kommt zur Bildung von Leerstellen. Interstitieller Einbau von Lithiumionen und Leerstellenbildung führen zu einem Anstieg der potentiellen Energie und der Konfigurationsentropie. Dies spiegelt sich in einer hohen Aktivierungsenergie und einer hohen Aktivierungsentropie für die Lithiumionenleitung in Li-TFSI wider.

Der Ersatz der TFSI-Anionen durch die weniger symmetrischen PFTFSI-Anionen führt zu einer geringeren Packungsdichte der Anionen und zu einer weniger günstigen Koordination der Lithiumionen. Die Lithiumionen in Li-PFTFSI besitzen daher eine höhere potentielle Energie und eine höhere Konfigurationsentropie im Vergleich zu Li-TFSI. Dies führt zu niedrigeren Werten der Aktivierungsenergie und - entropie für den Lithiumionentransport.

Die Ergebnisse der Impedanzspektroskopie für LiPFNFSI zeigen des Weiteren, dass es wegen der niedrigen Symmetrie der PFNFSI-Anionen keine eindeutige Unterscheidung zwischen Lithiumionen auf wohldefinierten Gitterplätzen und beweglichen Fehlstellen gibt, wie beispielsweise interstitiellen lonen und Leerstellen. Die Anzahl der verfügbaren Stellen für die Lithiumionenleitfähigkeit ist höher als die Anzahl der Lithiumionen selbst, was dazu führt, dass die Energie und Entropie für die Bildung beweglicher lonen vernachlässigbar ist.

Die lonenleitfähigkeit wurde für die Substanzen LiTFSI, Li-PFTFSI und Li-PFNFSI gemessen. Li-PFTFSI weist

die höchste Leitfähigkeit dieser drei Substanzen im gemessenen Temperaturfenster auf. Aus Fig. 7 ist ersichtlich, dass die Aktivierungsenergie $E_A^{act}$ für die Bildung beweglicher Lithiumionen in diesem Salz niedriger ist, als auf Grund einer Interpolation der Meyer-Neldel-Daten zwischen PFNFSI und LiTFSI zu erwarten war. Wenn also TFSI-Anionen durch PFTFSI-Anionen ersetzt werden, nimmt die Aktivierungsentropie wegen der niedrigeren Symmetrie der Anionen ab, doch die Aktivierungsenergie nimmt sogar in noch stärkerem Ausmaß ab, als anhand der Interpolation zu erwarten war. Die im Vergleich zu TFSI-Anionen niedrigere Symmetrie der PFTFSI-Anionen führt zu einem Anstieg der Konfigurationsentropie der Lithiumionen, doch der bedeutsamere Effekt ist die Abnahme der Aktivierungsenergie für die Leerstellenbildung. Die Koordination der Lithiumionen in Li-PFTFSI ist signifikant geringer als in Li-TFSI. Dennoch zeigt die vergleichsweise hohe Aktivierungsentropie, dass es in Li-PFTFSI einen eindeutigen Unterschied zwischen Lithiumionen auf regulären Gitterplätzen und mobilen Leerstellen gibt. Diese Art des Wechselspiels zwischen energetischen und entropischen Faktoren führt zu einer höheren Ionenleitfähigkeit von LiPFTFSI im Vergleich zu den beiden anderen Ionen.

Es ist für den Fachmann ohne Weiteres ersichtlich, dass ähnliche Aussagen in Bezug auf Symmetrie, Koordination der Lithiumionen, Ionenleitfähigkeit, Zunahme der Konfigurationsentropie und Abnahme der Aktivierungsenergie für die Leerstellen auch für andere erfindungsgemäße Lithiumsalze gelten.

Die erfindungsgemäßen Lithiumsalze können als Ionen leitende Materialien, elektrisch leitende Materialien, Farbstoffe und in der chemischen Katalyse verwendet werden. Bevorzugt werden sie als Ionen leitende Elektrolyte in Lithium-Ionenakkus verwendet. Lithium-Ionenakkus werden auch als Lithium-Ionenbatterien bezeichnet.

## Austührungsbeispiele

### Ausführungsbeispiel 1:

**Herstellung von Lithium-*bis*-pentafluorphenylamid Li-BPFPA (1)**

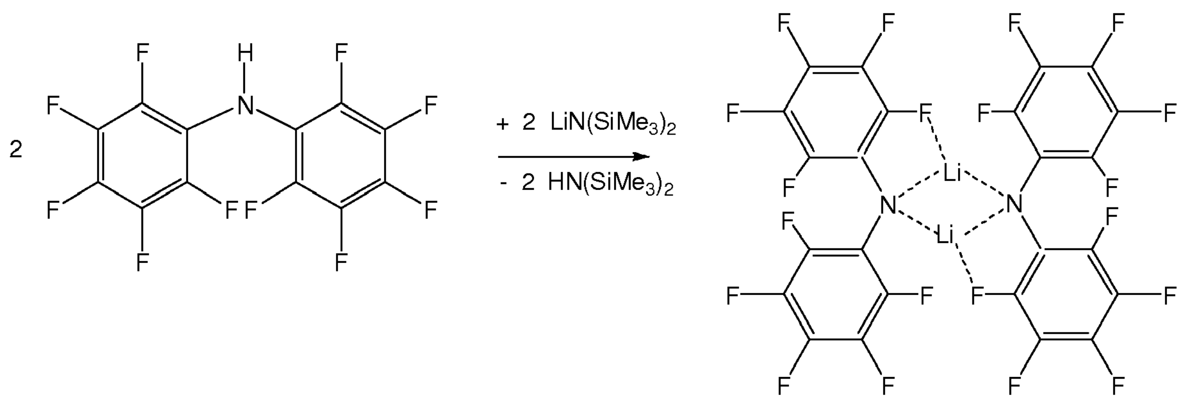


**1**

In einem Schlenk-Kolben wurden 0,97 g (2,77 mmol) *bis*-Pentafluorphenylamid (BPFPA-H) unter Erwärmen in 20 ml Toluol gelöst. Dann wurde eine Lösung von 0.47 g (2,77 mmol) $LiN(SiMe_3)_2$ in 10 ml Toluol über eine Spritze zugegeben. Die so erhaltene Suspension wurde für 12 h bei 80 ˚C gerührt. Alle flüchtigen Bestandteile wurden bei vermindertem Druck entfernt, der Rückstand mit Hexan gewaschen und im Vakuum getrocknet. Es wurden 0,54 g (55 %) 1 in Form eines farblosen Pulvers erhalten. Kristalle von röntgenographischer Qualität wurden aus einer gesättigten Toluollösung bei -30 ˚C erhalten.

$^{19}$F-NMR ([$D_6$]-DMSO, 282 MHz): δ = -185.7 nm (m, 2F, *p*-F), -170.10 (pt, 4F, *m*-F), -160.6 ppm (pd, 4F, o-F)
IR (Nujol): 570(w), 722(w), 823(w), 964(w), 999(m), 1031 (s), 1179(w), 1305(w) 1520 $cm^{-1}$(s)
ESI-MS (negativer Modus): m/z (%): 348.0 (100%) [$BPFPA^-$]
Elementaranalyse: ber. (%) für $C_{12}F_{10}LiN$: C 40.56, N 3.94; gef.: C 40.19, N 4.04

### Ausführungsbeispiel 2:

**Herstellung von 5,10-*bis*(Pentafluorphenyl)-5,10-dihydro-octafluorphenazin (2) durch unvorteilhafte**

**Thermolyse**

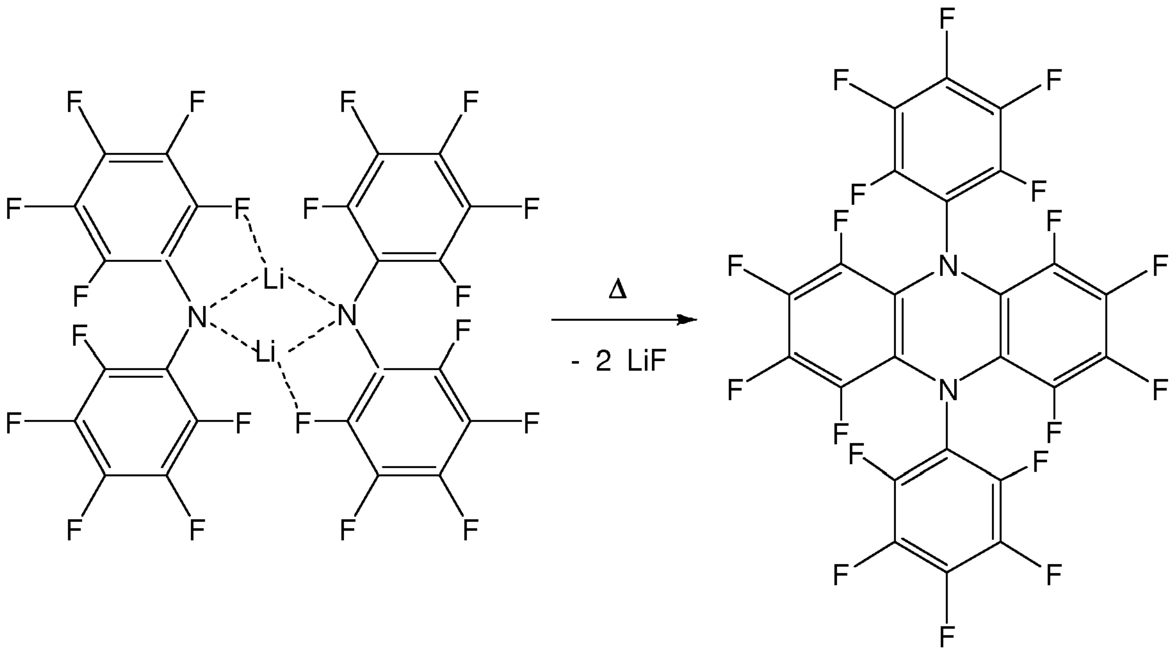


**2**

Ein Glasrohr wurde mit 0.50 g der Substanz **1** beladen und in einen elektrischen Ofen eingebracht. Das Rohr wurde an die Vakuumleitung angeschlossen ($10^{-2}$ mbar) und der Ofen langsam auf 220 ˚C erhitzt. Im kühleren Bereich des Rohres wurde ein gelbliches Sublimat beobachtet. Das Sublimat wurde gesammelt und mit warmem Hexan gewaschen, um durch BPFPA-H verursachte Verunreinigungen zu entfernen. Der so erhaltene farblose Feststoff wurde im Vakuum getrocknet. Es wurden 0,12 g der Verbindung **2** erhalten. Kristalle von röntgenographischer Qualität wurden aus einer Lösung in einem Gemisch von Hexan und Toluol bei -30 ˚C erhalten (ca. 1:4).

$^{19}$F-NMR ([$D_6$]-DMSO, 282 MHz): δ = -161.4 (pdt, 4F, m-F, $C_6F_5$), -160.8 (pdd, 4F, NCCFCF, Phenazin), -154.9 (pdd, 4F, NCC**F**, Phenazin), -149.6 (pt, 2F, *p*-F, $C_6F_5$), -141.6 ppm (ps, 4F, *o*-F, $C_6F_5$)
IR (KBr): 479(m), 608(m), 666(w), (684(w), 765(m), 822(m), 929(m), 995(m), 1067(s), 1097(s), 1166(w), 1190(w), 1260(w), 1321(w), 1443(m), 1509(s), 1619(s), 1637 $cm^{-1}$(s)
MS (70 eV): m/z(%): 658 (80%),[$M^+$], 491 (100%) [$M^+$-$C_6F_5$];
Elementaranalyse: ber. (%) für $C_{24}F_{18}LiN_2$: C 43.79, N 4.26; gef.: C 43.83, N 4.29

**Ausführungsbeispiel 3:**

**Herstellung von Lithium-pentafluorphenyltrifluormethylsulfonylimid Li-PFTFSI (3)**

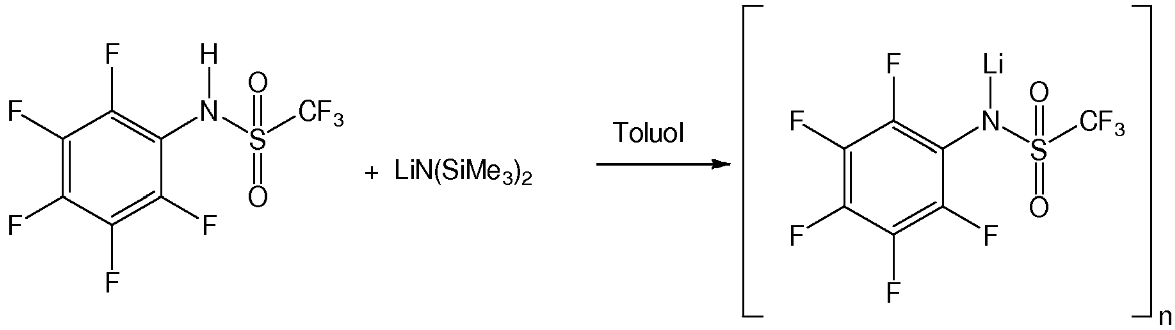


**3**

In einem Schlenk-Kolben wurden 0,49 g (1,54 mmol) Pentafluortrifluomethylsulfonylimid (PFTFSI-H) unter Erwärmen in 20 ml Toluol gelöst. Dann wurde eine Lösung von 0,25 g(1,54 mmol) $LiN(SiMe_3)_2$ in 10 ml Toluol über eine Spritze zugegeben. Die so erhaltene Suspension wurde für 12 h bei 80 ˚C gerührt. Alle flüchtigen Bestandteile wurden bei vermindertem Druck entfernt, und der Rückstand wurde mit Hexan gewaschen. Nach Trocknen im Vakuum wurde Verbindung **3** in Form eines farblosen Pulvers erhalten. Ausbeute: 0,44 g (88%).

$^{19}$F-NMR ([$D_6$]-DMSO, 282 MHz): δ = -168.2 (pt, 1 F, *p*-F), -166.8 (pt, 2F, *m*-F), - 146.1 (d, 2F, *o*-F), -77.5 ppm (t, 3F, $CF_3$);
IR (Nujol): 556(w), 608(w), 722(w), 801 (w), 909(m), 988(s), 1049(s), 1123(s), 1203(s), 1262(m), 1332(m), 1507 $cm^{-1}$(s)
ESI-MS (negativer Modus): m/z (%): 314.0 (100%) [$PFTFSI^-$];
Elementaranalyse: ber. (%) für $C_7F_8LiNO_2S$: C 26.17, N 4.36; gef.: C 25.41, N 4.82

**Ausführungsbeispiel 4:**

**Herstellung von Lithium-pentafluorphenylnonafluorbutylsulfonylimid Li-PFNFSI (4)**

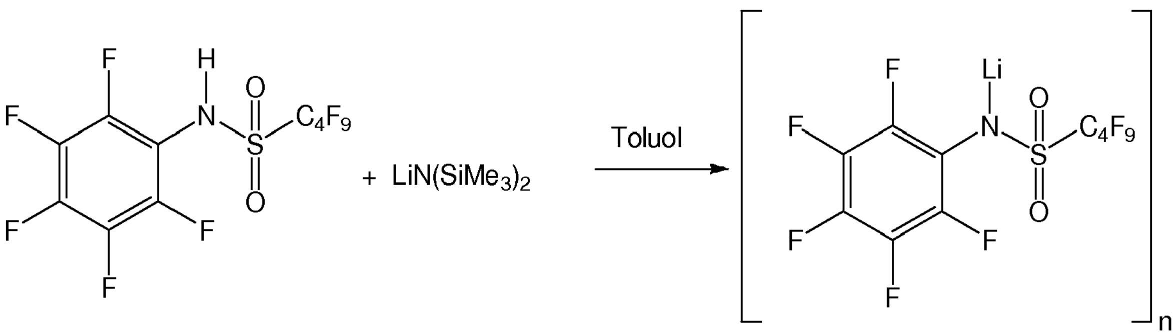


**4**

In einem Schlenk-Kolben wurden 1,11 g (2,38 mmol) Pentafluornonafluorbutylsulfonylimid (PFNFSI-H) unter Erwärmen in 20 ml Toluol gelöst. Dann wurde eine Lösung von 0,40 g (2,38 mmol) $LiN(SiMe_3)_2$ in 10 ml Toluol über eine Spritze zugegeben. Die so erhaltene Suspension wurde für 12 h bei 80 ˚C gerührt. Alle flüchtigen Bestandteile wurden bei vermindertem Druck entfernt, und der Rückstand wurde mit Hexan gewaschen. Nach Trocknen im Vakuum wurde Verbindung **4** in Form eines farblosen Pulvers erhalten. Ausbeute: 0,99 g (88%). $^{19}$F-NMR ([$D_6$]-DMSO, 282 MHz): δ = -168.2 (pt, 1 F, p-F), -166.8 (pt, 2F, m-F), -149.8 (pd, 2F, o-F), -125.8 (s, 2F, $SO_2CF_2$), -120.8 (t, 2F, $SO_2CF_2CF_2$), -114.1 (t, 2F, $F_3CCF_2$), -80.4 ppm (t, 3F, $CF_3$);

IR (Nujol): 501 (w), 593(m), 722(w), 784(w), 901 (m), 989(s), 1056(s), 1136(s), 1060(s), 1211 (s), 1310(s), 1518 $cm^{-1}$(s)
ESI-MS (negativer Modus): m/z (%): 464.0 (100%) [$PFNFSI^-$]

Elementaranalyse: ber. (%) für $C_{10}F_{14}LiNO_2S$: C 25.48, N 2.97; gef.: C 24.40, N 2.63

**Ausführungsbeispiel 5:**

**Herstellung von des THF-Komplexes von Lithium-pentafluorphenylnonafluorbutylsulfonylimid [Li(THF)$_2$(PFNFSI)] 4(THF)$_2$**

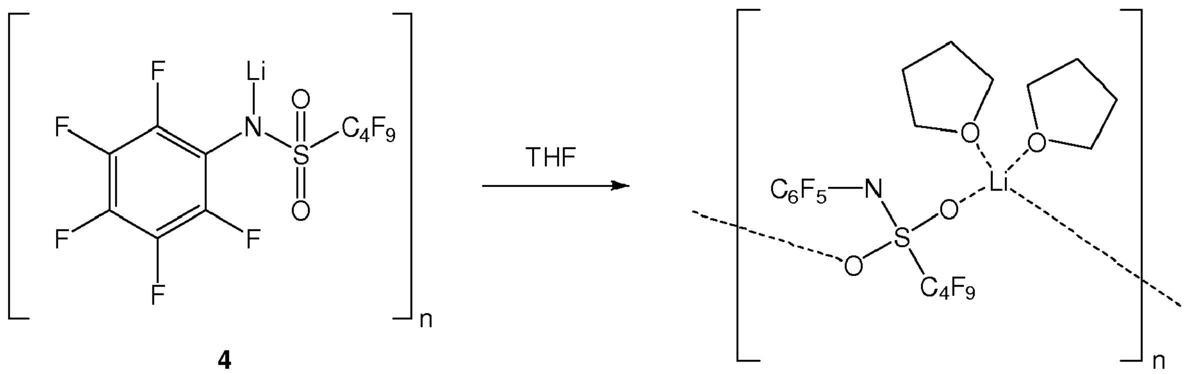


**4** **4(THF)$_2$**

Die Umkristallisation von Verbindung **4** aus Ausführungsbeispiel 4 in THF bei - 30 ˚C ergibt farblose Kristalle von [Li(THF)$_2$(PFNFSI)], die für die Röntgenstrukturanalyse geeignet sind.

**Röntgenkristallstrukturanalyse**

Die Röntgendiffrationsintensitäten wurden auf einem STOE IPDS-I- und IPDS-II-Diffraktometersystem unter Verwendung von Mo-$K_\alpha$-Strahlung (0.71073 Å) gemessen. Die Strukturen wurden mit Hilfe direkterMethoden bestimmt, mit Hilfe einer daran anschließenden Differenzfouriersynthese vervollständigt und nach der Methode der kleinsten Quadrate im Vollmatrix-Verfahren verfeinert. Alle Nichtwasserstoffatome wurden mit anisotropen Auslenkungskoeffizienten verfeinert. Die Wasserstoffatome wurden berechnet und isotrop verfeinert. Folgende Programme wurden verwendet: WinGX, SIR-97, SHELXL-97.

**Ausführungsbeispiel 6:**

**Röntgenkristallstrukturanalyse von Lithium-bis-pentafluorphenylamid Li(BPFPA) (1)**

Li(BPFPA) **1** kristallisiert in der Raumgruppe P $2_1$/c mit Z = 8. Im festen Zustand bildet es ein Dimer. Die beiden Lithiumatome sind über die beiden Stickstoffatome der verbrückenden Amidoliganden unter Ausbildung einer fast planaren $Li_2N_2$-Einheit verbunden. Jedes Stickstoffatom bildet eine kürzere (2.08 Å) und eine längere (2.10 Å bzw. 2.11 Å) Li-N-Bindung aus. Diese Bindungslängen sind innerhalb von 3σ identisch mit den Strukturen der Etherate. Im Gegensatz zu den Etheraten vervollständigen die Lithiumatome in 1 ihre Koordinationssphären jedoch exklusiv mit einem Satz schwacher Kontakte zu Fluoratomen. Jedes Lithiumatom ist über zwei Stickstoffatome und vier Fluoratome sechsfach koordiniert. Die vier intramolekularen Li-F-Kontakte des Dimers in Fig. 1 sind kurz und variieren im Bereich von 2.02 Å bis 2.22 Å. Einer der beiden kurzen Li-F-Kontakte jedes Lithiumatoms stammt aus den *ortho*-Fluoratomen der Pentafluorphenyl-Einheit (Pfp) eines Amidoliganden, wohingegen der andere durch das *ortho*-Fluoratom der Pfp-Einheit des benachbarten Amidoliganden der dimeren Einheit ausgebildet wird. In dieser Hinsicht kann der Ligand BPFPA als a $\kappa^3$-(N,F,F)-verbrückende Einheit betrachtet werden. Die intermolekularen Li-F-Kontakte vervollständigen die verzerrt oktaedrische Koordination des Lithiums und sind im Wesentlichen in der äquatorialen $Li_2N_2$-Ebene orientiert. Sie sind etwas länger als die intramolekularen Abstände und weisen Kontaktabstände bis zu 2.60 Å bzw. 2.67 Å auf. Als Folge der intermolekularen Li-F-Kontakte wird ein dreidimensionales Netzwerk gebildet, in dem jede dimere Einheit über acht koordinative Bindung mit vier benachbarten Einheiten koordiniert ist.

**Ausführungsbeispiel 7:**

**Röntgenkristallstrukturanalyse von 5,10-*bis*(Pentafluorphenyl)-5,10-dihydrooctafluorphenazin (2)**

5,10-*bis*(Pentafluorphenyl)-5,10-dihydro-octafluorphenazin **2** kristallisiert in der Raumgruppe P 1 with Z = 1. Das Molekül weist eine kristallographisch bedingte Inversionssymmetrie auf. Die Molekülstruktur von **2** weist eine planare Anordnung der Atome der zentralen Dihydrophenazineinheit auf. Es wurde gefunden, dass der Winkel zwischen den beiden Ebenen, die durch die beiden Arylringe definiert wird, ein Idealwinkel von 180° ist. Dies steht im Gegensatz zu den nicht fluorierten Derivaten, bei denen die Dihydrophenazineinheit in der N-N-Achse gefaltet ist, wie beispielsweise in 5,10-Dimethyl-5,10-dihydrophenazin, das einen interplanaren Winkel von 144°aufweist, und in 2,7-Dibrom-1,6-dichlor-5,10-dimethyl-5,10-dihydrophenazin, dessen interplanarer Winkel 152° beträgt. Aus sterischen Gründen und als Konsequenz der Planarität des zentralen Rings weichen die Pfp-Substituenten am Stickstoffatom von der Ebene ab und nehmen eine *anti*-Anordnung in Bezug aufeinander an. Der Winkel zwischen der C7-N1-Bindung und der Ebene des Dihydrophenazins wurde zu 41 ° ermittelt. Die Bindungslängen der C-N-Bindungen in 2 wurden zu 1.41 Å und 1.43 Å bestimmt und liegen in derselben Größenordnung wie in den nicht fluorierten Dihydrophenazinen, für die in der Literatur Werte von 1.41 Å für 5,10-Dimethyl-5,10-dihydrophenazin sowie Werte zwischen 1.37 Å und 1.39 Å für 2,7-Dibrom-1,6-dichlor-5,10-dimethyl-5,10-dihydrophenazin berichtet wurden.

Zusammengefasst weisen die Eigenschaften des perfluorierten Dihydrophenazins eine große strukturelle Differenz zu den nicht fluorierten, elektronenreichen Stammverbindungen auf.

**Ausführungsbeispiel 8:**

**Röntgenkristallstrukturanalyse der Verbindungen (3) und (4)**

Da die Verbindungen **3** und **4** in nicht koordinierenden Lösungsmitteln nahezu unlöslich sind, wurden sie unter Verwendung von THF umkristallisiert.

Im Falle von **4** wurden Kristalle von röntgenographischer Qualität aus einer THF-Lösung bei -30 °C erhalten werden. Die Strukturanalyse offenbart eine Struktur, die zwei THF-Moleküle pro Li(PFNFSI) enthält, siehe Ausführungsbeispiel 5. **4**$(THF)_2$ kristallisiert in der Raumgruppe P 1 mit Z = 1 und bildet eine Kettenstruktur, in der die Lithiumatome exklusiv durch Sauerstoffatome koordiniert werden; es konnten keinerlei Li-N- und Li-F-Kontakte beobachtet werden. Alle Lithium-Kationen werden durch zwei Sauerstoffatome der Sulfonylgruppen der PFNFSI-Anionen und zwei Sauerstoffatome der THF-Moleküle tetraedrisch koordiniert. Für die Abstände zwischen den Lithium- und den Sauerstoffatomen wurden Werte zwischen 1.92 und 1.96 Å ermittelt. Diese Abstände sind geringfügig kürzer als in der strukturell verwandten Verbindung $[LiN(SO_2CF_3)_2(DME)]$. Dabei steht DME für Dimethoxyethan. Da keine besonders kurzen Li-O-Abstände ermittelt wurden, gibt es nur schwache Wechselwirkungen zwischen Kation und Anion. Das Stickstoffatom, welches formal die negative Ladung trägt, zeigt keine Wechselwirkung mit dem Kation. Tatsächlich findet die Delokalisation der negativen Ladung im Wesentlichen über die Sulfonyleinheit statt. Der ermittelte vergleichsweise kurze S-N-Abstand von 1.52 Å, der im Bereich einer S-N-Doppelbindung liegt, befindet sich in Übereinstimmung mit dieser Delokalisierung. Im Vergleich zur Referenzverbindung $[LiN(SO_2CF_3)_2(DME)]$ (1.57 Å) ist die S-N-Bindungslänge in **4**$(THF)_2$ kürzer, da die negative Ladung am Stickstoffatom im Falle von **4**$(THF)_2$ in stärkerem Ausmaß in die THF-Gruppe als in die Pentafluorphenyl-Gruppe delokalisiert wird. Auch der Bindungswinkel am Stickstoffatom weist mit einem Wert von 121 ° auf einen S-N-Doppelbindungscharakter hin.

**Ausführungsbeispiel 9:**

**Hydrolyse der Verbindung (1)**

Die Stabilität von **1** gegenüber Hydrolyse wurde untersucht. Eine Lösung von **1** in $[D_6]$-DMSO ergibt ein Signal bei $\delta_F$ = -185.7 ppm für das *para*-Fluoratom im 282 MHz $^{19}F$-NMR-Spektrum, wohingegen dasselbe Fluoratom im freien Amin zum tiefen Feld verschoben ist bei $\delta_F$ = -165.5 ppm.

Es wurde versucht, 1 durch Zugabe von Wasser in das freie Amin zu überführen. Es konnte gezeigt werden, dass die Zugabe von Wasser zu **1** in $[D_6]$-DMSO zu keinerlei Bildung des freien Amins führte. Freie Amine sind die zuerst entstehenden Hydrolyseprodukte eines Metallamids und wären mit den verwendeten Analysemethoden nachweisbar gewesen. Der $pK_a$-Wert von H-BPFPA beträgt in DMSO bei 25°C 12,6. Um diese vergleichsweise stark NH-acidische Verbindung herzustellen, wird üblicherweise ein korrespondierendes Salz mit einer starken Säure wie beispielsweise wässriger HCl behandelt. Verbindung 1 ist wenig hygroskopisch, doch bei Raumtemperatur wird keine weitere Hydrolyse beobachtet.

**Ausführungsbeispiel 10:**

**Hydrolyse der Verbindungen (3) und (4)**

Wie in Ausführungsbeispiel 9 für Verbindung 1 beschrieben, wurde auch die Stabilität der Verbindungen 3 und 4 gegenüber Hydrolyse mit Hilfe der $^{19}$F-NMR-Spektroskopie durch Zugabe von Wasser zu einer [$D_6$]-DMSO-Lösung von **3** und **4** gezeigt.

Während das Signal für die para-Fluoratome des Pfp-Restes in den Anionen bei $\delta_F$ = -168.2 ppm (3) und $\delta_F$ = -168.2 ppm (**4**) erscheint, werden im NMR keinerlei Spuren der korrespondierenden Sulfonamidsäuren bei $\delta_F$= -163.9 ppm (H-PFTFSI) und $\delta_F$ = -165.8 ppm (H-PFNFSI) gefunden.

**Thermogravimetrische Analyse (TGA) und Differential Scanning Calorimetry (DSC)**

Thermogravimetrische Analysen wurden mit einem METTLER Toledo TGA/SDTA 851 $^e$ unter einem konstanten Stickstoffstrom in Aluminiumtiegeln durchgeführt. Differential Scanning Calorimetry-Messungen wurden mit einem METTLER Toledo DSC 821 unter einem konstanten Stickstoffstrom in offenen Aluminiumtiegeln durchgeführt.

**Ausführungsbeispiel 11:**

**Thermogravimetrische Analyse (TGA) und Differential Scanning Calorimetry (DSC) der Verbindung (1)**

Die thermische Stabilität der Verbindung 1 wurde mittels thermogravimetrischer Analyse (TGA) und Differential Scanning Calorimetry (DSC) untersucht.

Die TGA zeigt einen Beginn des Masseverlustes bei 108 ˚C. Bei 180˚ wurde ein starker Masseverlust bis auf 15 % der ursprünglichen Masse verzeichnet (Fig. 4). Die DSC ergibt in der Heizphase einen Glasübergang bei -31 ˚C und einen endothermen Phasenübergang bei 87 ˚C mit einer Übergangsenthalpie von 23,8 kJ/mol.

**Ausführungsbeispiel 12:**

**Thermogravimetrische Analyse (TGA) und Differential Scanning Calorimetry (DSC) der Verbindungen (3) und (4)**

Die thermische Stabilität der Verbindungen **3** und **4** wurde mittels thermogravimetrischer Analyse (TGA) und Differential Scanning Calorimetry (DSC) untersucht. Es wurde gefunden, dass das Einführen einer Triflyl-Gruppe (Tf, $CF_3$) oder einer Nonaflyl-Gruppe (Nf, $C_4F_9$) großen Einfluss auf die thermische Stabilität der entsprechenden Lithiumsalze hat.

Verbindung **3** beginnt sich bei einer Temperatur von 307 ˚C zu zersetzen (0,05% Gewichtsverlust), während die Zersetzung bei Verbindung **4** bei 316 ˚C beginnt. Diese thermische Stabilität ist ausreichend für die gewünschten elektrochemischen Anwendungen.

Zuletzt wurden DSC-Messungen bis 10 ˚C unter den Zersetzungspunkt der beiden Substanzen durchgeführt, wobei in beiden Fällen lediglich Glasübergänge stattfanden. Verbindung **3** zeigt bei -8 ˚C einen Glasübergang, während Verbindung **4** den Glasübergang bei 22 ˚C zeigt.

Die Ergebnisse der TGA-Messungen sind in Fig. 4 gezeigt.

**Ausführungsbeispiel 13:**

**Impedanzspektroskopische Messungen**

Impedanzspektroskopische Messungen wurden an den festen Lithiumsalzen Li-PFTFSI und Li-PFNFSI durchgeführt, um Informationen über deren LithiumIonenleitfähigkeitseigenschaften im festen Zustand zu erhalten. Zum Vergleich wurden ebenfalls Impedanzspektren des bekannten Salzes Li-TFSI aufgenommen. Aus der frequenzabhängigen komplexen Impedanz $\hat{Z}(\nu)$ =Z'($\nu$) -*i*.Z"($\nu$) wurde der Realanteil der Leitfähigkeit, σ'($\nu$) , berechnet gemäß:

$$\sigma'(\nu) = \frac{d}{A} \cdot \frac{Z'(\nu)}{[Z'(\nu)]^2 + [Z''(\nu)]^2} \qquad (1)$$

Hier bezeichnen *d* und *A* die Dicke bzw. die Fläche der Probe.

In Fig. 5 sind σ'(*v*) -Spektren für Li-PFTFSI bei verschiedenen Temperaturen gezeigt. Zwischen 50 ˚C und 100 ˚C ist der Niederfrequenzbereich des Spektrums durch einen Plateaubereich charakterisiert. In diesem Bereich ist σ'(*v*) identisch mit der Volumenleitfähigkeit bei Gleichstrom (bulk dc conductivity) $\sigma_{dc}$, was den makroskopischen Lithiumionentransport widerspiegelt. Bei höheren Frequenzen wird σ'(*v*) frequenzabhängig. Dieser dispersive Bereich enthält zusätzliche Informationen über lokalisierte Bewegungen der Lithiumionen in den Salzen. Bei den beiden höchsten Temperaturen sind die Spektren unter 10 Hz durch einen Abfall von σ'(*v*) bei abnehmender Frequenz gekennzeichnet. Daher ist das Plateau der Volumenleitfähigkeit nur bei Frequenzen über 10 Hz detektierbar.

Fig. 6 zeigt einen Arrhenius-Plot der Volumenleitfähigkeit bei Gleichstrom (bulk dc conductivity) $\sigma_{dc}$, aller Lithiumsalze. Im untersuchten Temperaturbereich ergab sich die höchste Leitfähigkeit für Li-PFTFSI, die zweithöchste für Li-TFSI und die niedrigste für Li-PFNFSI. Die Daten wurden gemäß dem Arrhenius-Gesetz angepasst:

$$\sigma_{dc} \cdot T = A \cdot \exp\left(\frac{E_A}{k_B T}\right) \qquad (2)$$

Hier bezeichnen *A* und $E_A$ den präexponentiellen Faktor bzw. die Aktivierungsenergie der DC-Leitfähigkeit (Gleichstromleitfähigkeit), wobei $k_B$ die Boltzmann-Konstante ist.

Die Aktivierungsenergie nimmt in der Reihenfolge $E_A$ (Li-TFSI) > $E_A$ (Li-PFTFSI) > $E_A$ (Li-PFNFSI ab. Bemerkenswerterweise weist Li-PFNFSI, also das Salz mit der niedrigsten Leitfähigkeit, auch die niedrigste Aktivierungsenergie auf.

In Fig. 7 ist der präexponentielle Faktor A logarithmisch gegen die Aktivierungsenergie $E_A$ aufgetragen. Es ist offensichtlich, dass der präexponentielle Faktor mit steigender Aktivierungsenergie ebenfalls ansteigt. Diese Eigenschaft stimmt mit der bekannten Meyer-Neldel-Regel überein (MN-Regel). Diese Regel besagt, dass für eine Familie ionenleitfähiger Materialien mit ähnlichen Strukturen gilt:

$$\log(A) = \alpha \cdot E_A + \beta \qquad (3)$$

Zwei bekannte Familien von Lithium-Ionenleitern sind in Fig. 7 enthalten: polykristalline LISICON-Materialien der Zusammensetzung $Li_{2+2x}Zn_{1-x}GeO_4$ und LISICON-artige Gläser der Zusammensetzung $Li_{2.6+x}Ti_{1.4-x}Cd(PO_4)_{3\cdot 4-x}$. Beide Familien werden durch die MN-Regel gut beschrieben.

Vom theoretischen Standpunkt aus betrachtet ist zu erwarten, dass Materialien der MN-Regel folgen, wenn die Mehrheit der Ionen in tiefen Potentialen gefangen ist, so dass thermische Aktivierung benötigt wird, um mobile Ionen zu erzeugen. Ein Beispiel ist die Bildung von mobilen interstitiellen Ionen und Leerstellen in Kristallen. In diesem Fall kann die Aktivierungsenergie $E_A$ als die Summe zweier Terme geschrieben werden:

$$E_A = E_A^{act} + E_A^{mig} \qquad (4)$$

Der erste Term $E_A^{act}$ ist die zur thermischen Aktivierung der gefangenen Ionen erforderliche Energie, wohingegen der zweite Term $E_A^{mig}$. die Aktivierungsenergie für die Migration der aktivierten Ionen beschreibt (z.B. Migration von Defekten in Kristallen). Die Aktivierung gefangener Ionen wird auch durch eine Aktivierungsentropie $S_A^{act}$ charakterisiert, die in den präexponentiellen Faktor *A* eingeht:

$$A = \frac{N_v \cdot e^2 \cdot a^2 \cdot v_0}{6k_B} \cdot \exp\left(\frac{S_A^{act}}{k_B}\right) = A_0 \cdot \exp\left(\frac{S_A^{act}}{k_B}\right) \qquad (5)$$

Hier bezeichnen $N_v$ und *a* die Teilchenzahldichte aller Ionen in der Probe bzw. die Sprungdistanz, während *e* die Elementarladung ist. Die Versuchsfrequenz $v_0$ kann mit der Schwingungsfrequenz der Ionen in ihren Potentialkäfigen gleichgesetzt werden. Einsetzen typischer Werte für diese Größen führt zu $log(A_0.\ \Omega cm/K) \approx 5$.

Die Meyer-Neldel-Regel basiert nun auf der Annahme, dass

$$S_A^{act} = \frac{E_A^{act}}{T_0} \qquad (6)$$

Diese Gleichung impliziert, dass bei der Temperatur $T_0$ die freie Energie für die Aktivierung getrappter Ionen Null wird. Aus den Gleichungen (4) - (6) folgt, dass

$$\log(A) = \log(A_0) + \frac{E_A - E_A^{mig}}{kT_0 \cdot \ln(10)} \qquad (7)$$

In Übereinstimmung mit der MN-Regel (3) kann Gleichung (7) verwendet werden, um die Parameter $E_A^{mig}$ und $T_0$ aus experimentellen Daten zu bestimmen.

Während die Daten der erfindungsgemäßen Verbindungen der MN-Regel in qualitativer Weise folgen, weisen die Daten von log(*A*) gegen $E_A$ keine lineare Beziehung auf. Dies impliziert, dass die Ähnlichkeiten in der Struktur und in den Mechanismen der Ionenleitung nicht ausreichen, um ein elektrochemisches Verhalten hervorzubringen, das exakt der Meyer-Neldel-Regel folgt. Daher kann keine genaue Information über $E_A^{mig}$ und $T_0$ gewonnen werden. Gleichung (5) kann jedoch zur Berechnung der Aktivierungsentropie $S_A^{act}$ verwendet werden. Es wurden Werte von $S_A^{act} = 24 \cdot k_B$ für Li-PFTFSI und $S_A^{act} = 34{,}5 \cdot k_B$ für Li-TFSI erhalten. Im Falle von Li-PFNFSI sind Energie und Entropie für die Aktivierung von Lithiumionen vernachlässigbar.

**Abbildungslegenden**

**Fig. 1**

ORTEP-Ausdruck der Verbindung **1**, 30 % Wahrscheinlichkeit der Ellipsoide. Ausgewählte Bindungslängen [Å] und Winkel [˚]:

Li1-N001 2.101 (5), Li1-N101 2.084(5), Li2-N001 2.082(5), Li2-N101 2.110(5),
F001-Li1 2.050(4), F105-Li1 2.224(4), F102"-Li1 2.032(5), F005"-Li1 2.677(5),
F107-Li2 2.022(4), F010-Li2 2.152(4), F007'-Li2 2.043(4), F111'-Li2 2.605(5),
N101-Li1-N001 102.9(2), N001-Li2-N101 102.7(2), Li1-N101-Li2 77.0(2),
Li2-N001-Li1 77.3(2).

**Fig. 2**

ORTEP-Ausdruck der Verbindung **2**, 30 % Wahrscheinlichkeit der Ellipsoide. Ausgewählte Bindungslängen

[Å] und Winkel [˚]:

C1-C2 1.407(5), C1-N1 1.432(4), N1-C2 1.414(5), C7-N1 1.465(4), C2-N1-C1 115.6(3), C1-N1-C7 112.8(3), C2-N1-C7 113.7(3).

**Fig. 3**

ORTEP-Ausdruck der Verbindung **4**(**THF**)$_2$, 30 % Wahrscheinlichkeit der Ellipsoide.
Ausgewählte Bindungslängen [Å] und Winkel [˚]:

Li1-01 1.917(7), Li1-02' 1.957(7), Li1-03 1.920(5), Li1-04 1.926(8), S1-N1 1.521 (3), S1-O1 1.438(3), S1-O2 1.450 (3), O1-S1-O2 116.0(2), O1-S1-N1 111.3(2), O2-S1-N1 116.7(2).

**Fig. 4**

Thermogravimetrische Analyse der Verbindungen 1, 3 und 4.

Durchgezogene Linie: Li-BPFPA (**1**)
Gestrichelte Linie: Li-PFTFSI (**3**)
Gepunktete Linie: Li-PFNFSI (**4**)

**Fig. 5**

Leitfähigkeitsspektren von festem Li-PFTSI (3) bei verschiedenen Temperaturen.

**Fig. 6**

Arrhenius-Plot der Leitfähigkeit verschiedener Lithiumsalze.

**Fig. 7**

Meyer-Neldel-Plot der präexponentiellen Faktoren und Aktivierungsenergien. Daten für kristalline und glasartige LISICON-artige Materialien aus dem Stand der Technik werden zum Vergleich gezeigt.

**Fig. 8**

Cyclovoltammogramm von Li-PFTFSI, gemessen an einer Platin-Arbeitselektrode gegen eine Silber-Referenzelektrode mit einer Vorschubsgeschwindigkeit von 20 mV/s. Das elektrochemische Fenster beträgt 4,2 V.
EC/DMC bedeutet Ethylencarbonat / Dimethylcarbonat.

**Fig. 9**

Cyclovoltammogramm von Li-PFNFSI, gemessen an einer Platin-Arbeitselektrode gegen eine Silber-Referenzelektrode mit einer Vorschubsgeschwindigkeit von 20 mV/s. Das elektrochemische Fenster beträgt 4,2 V.

**Fig. 10**

Cyclovoltammogramm von Li-TFSI , gemessen an einer Platin-Arbeitselektrode gegen eine Silber-Referenzelektrode mit einer Vorschubsgeschwindigkeit von 20 mV/s. Das elektrochemische Fenster beträgt 4,9 V.

## Patentansprüche

1. Pentafluorphenylamidoanionen umfassende Lithiumsalze gemäß Formel (I)

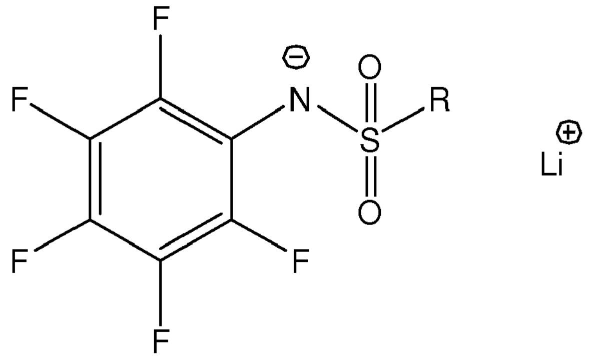

(I)

,

worin R ausgewählt ist aus

- F,
- linearen oder verzweigten acyclischen oder cyclischen Alkylgruppen mit 1 bis 20 Kohlenstoffatomen, die nicht fluoriert, partiell fluoriert oder vollständig fluoriert sind,
- nicht fluorierte, partiell fluorierte oder vollständig fluorierte Aryl- oder Benzylgruppen mit bis zu 20 Kohlenstoffatomen.

**2.** Verbindung gemäß Anspruch 1, **dadurch gekennzeichnet, dass** R ausgewählt ist aus der Gruppe Fluor, Trifluormethyl und Nonafluor-n-butyl.

**3.** Verbindung gemäß einem der Ansprüche 1 oder 2, **dadurch gekennzeichnet, dass** kein Lösungsmittelmolekül an der Koordination des Lithiumatoms beteiligt ist.

**4.** Verfahren zur Herstellung von Verbindungen gemäß Anspruch 1, umfassend die Schritte:

a) Lösen der freien Säure des Pentafluorphenylamids gemäß Formel II

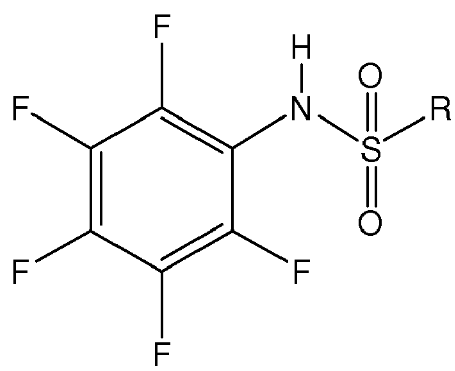

(II)

in einem unpolar aprotischen oder dipolar aprotischen Lösungsmittel unter Erwärmen,
b) Mischen mit einer äquimolaren Menge von Lithiumbistrimethylsilylamid oder eines Lithiumorganyls als Feststoff oder in Lösung,
c) Einstellen des so erhaltenen Gemisches aus freier Säure des Pentafluorphenylamids und des Lithiumtrimethylsilylamids oder des Lithiumorganyls auf eine Gesamtkonzentration dieser beiden Reaktanden von 0,01 mol/L bis 0,5 mol/L,
d) Rühren bei 80 ˚C für 12 Stunden,
e) Abkühlen des Reaktionsgemisches auf Raumtemperatur,
f) Entfernen der flüchtigen Bestandteile des Gemisches,
g) Waschen des Rückstandes mit einem unpolar aprotischen oder dipolar aprotischen Lösungsmittel und anschließendes Trocknen.

**5.** Verfahren gemäß Anspruch 4, **dadurch gekennzeichnet, dass** das Lithiumbistrifluorsilylamid oder das Lithiumorganyl gemäß Schritt b) in Lösung zugegeben wird.

**6.** Verfahren gemäß Anspruch 5, **dadurch gekennzeichnet, dass** das Mischen gemäß Schritt b) erfolgt, indem eine Lösung des Lithiumbistrimethylsilylamids oder des Lithiumorganyls vorgelegt und dann die Lösung des Pentafluorphenylamids gemäß Schritt a) zugegeben wird.

**7.** Verfahren gemäß Anspruch 4, **dadurch gekennzeichnet, dass** das Lithiumbistrifluorsilylamid oder das Lithiumorganyl gemäß Schritt b) als Feststoff zugegeben wird.

**8.** Verfahren gemäß einem der Ansprüche 5 bis 7, **dadurch gekennzeichnet, dass** es unter Verwendung unpolar aprotischer Lösungsmittel durchgeführt wird.

**9.** Verwendung von Verbindungen gemäß einem der Ansprüche 1 bis 3 als Ionen leitende Elektrolyte in Lithium-Ionenakkus.

**Fig. 1**

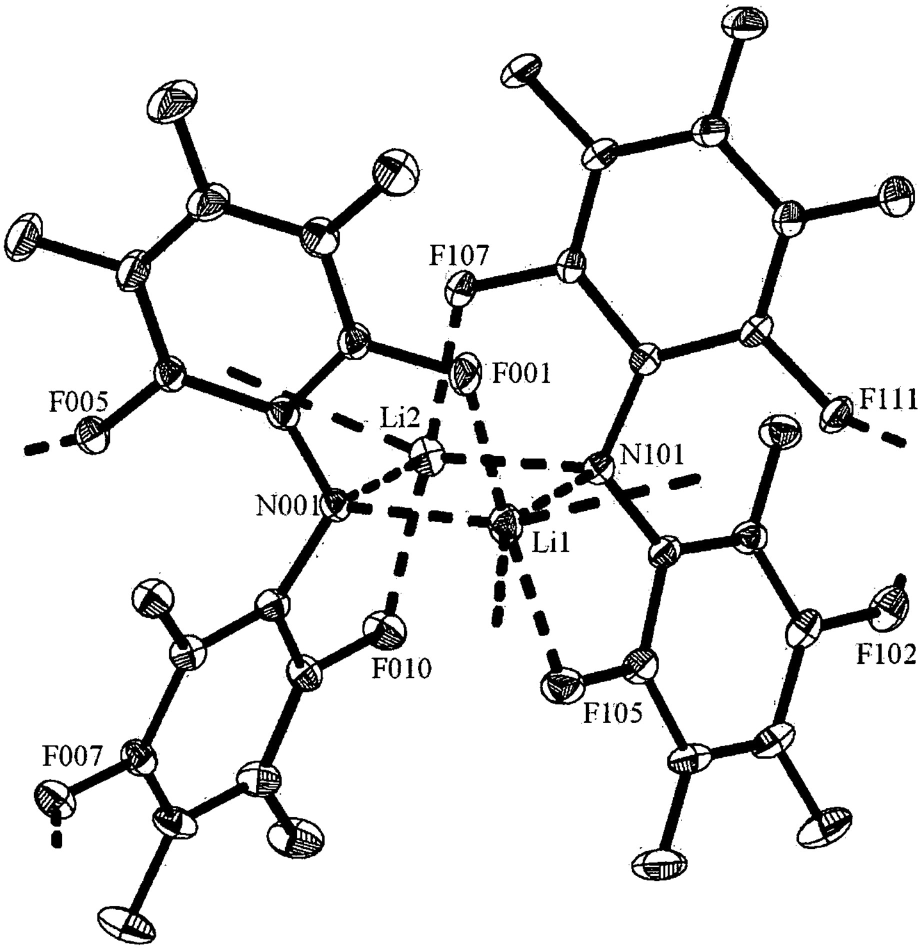

**Fig. 2**

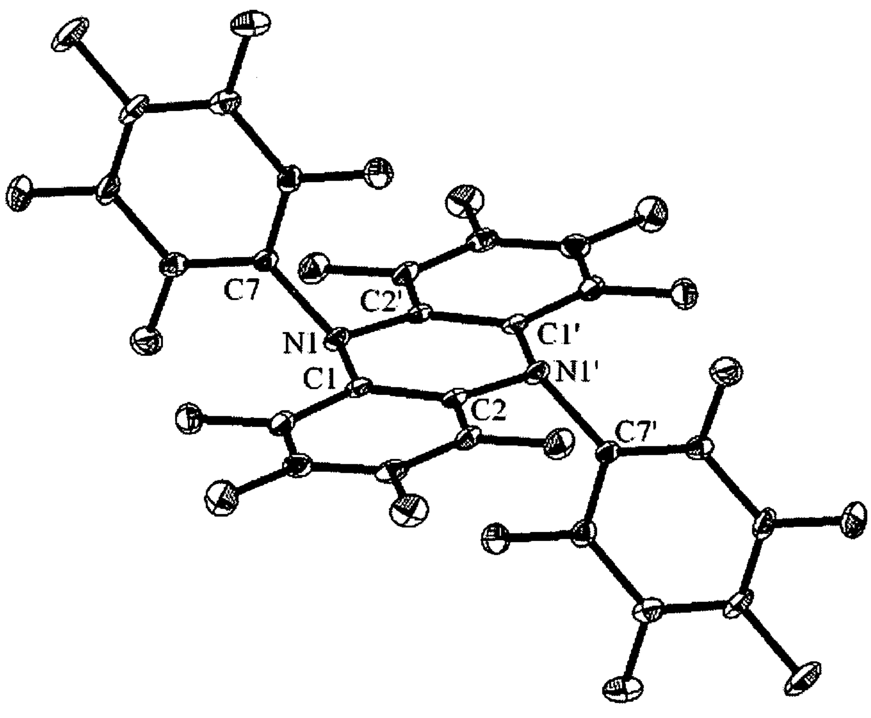

Fig. 3

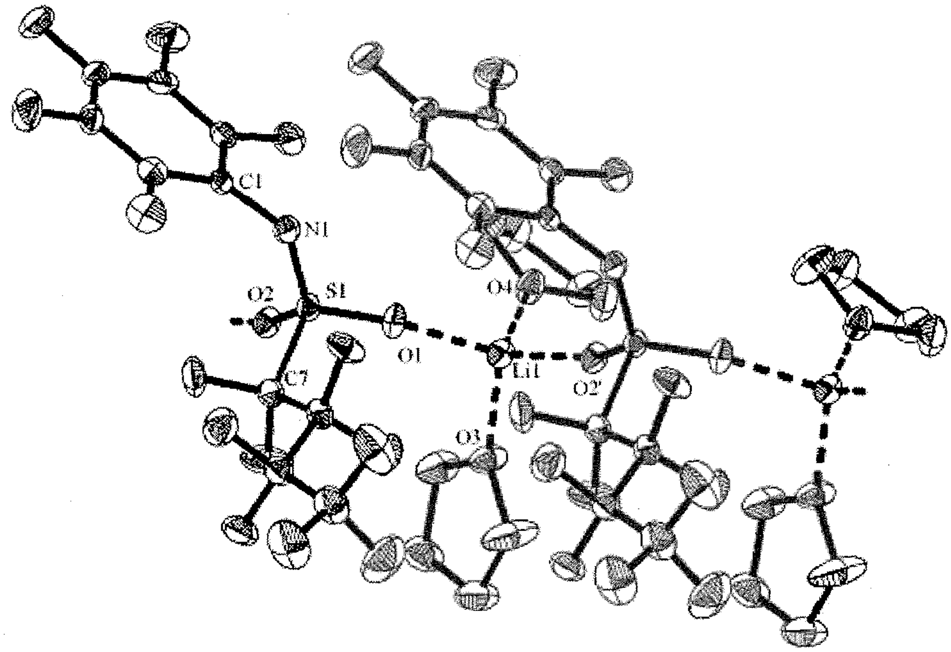

Fig. 4

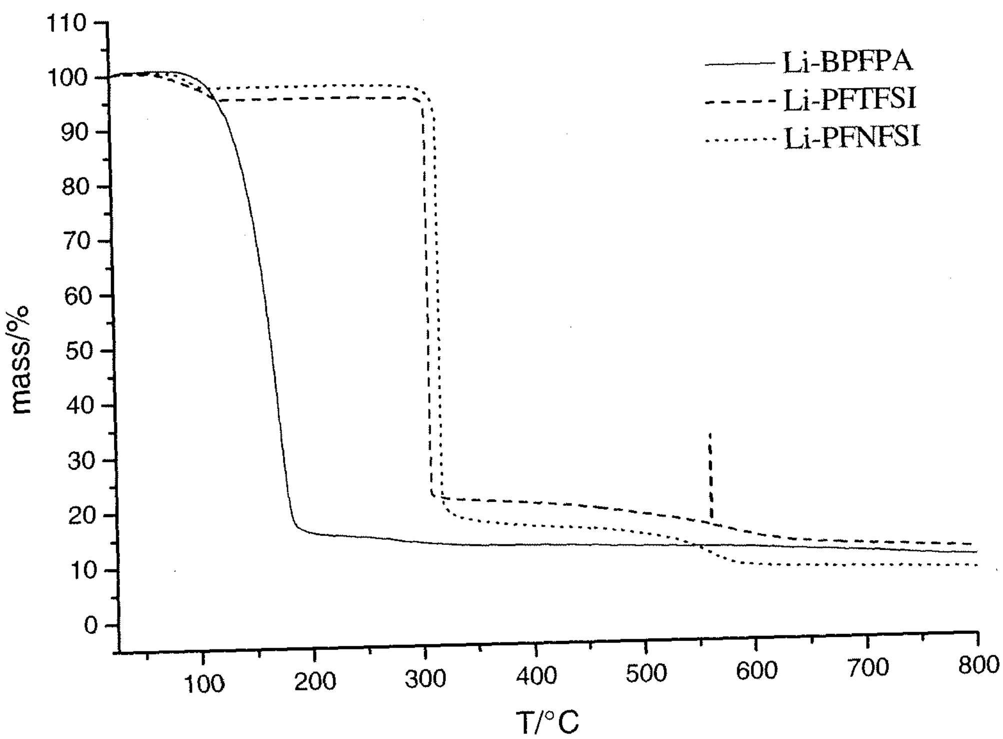
110
100
90
80
70
60
50
40
30
20
10
0
mass/%
100
200
300
400
500
600
700
800
T/°C
Li-BPFPA
Li-PFTFSI
Li-PFNFSI

Fig. 5

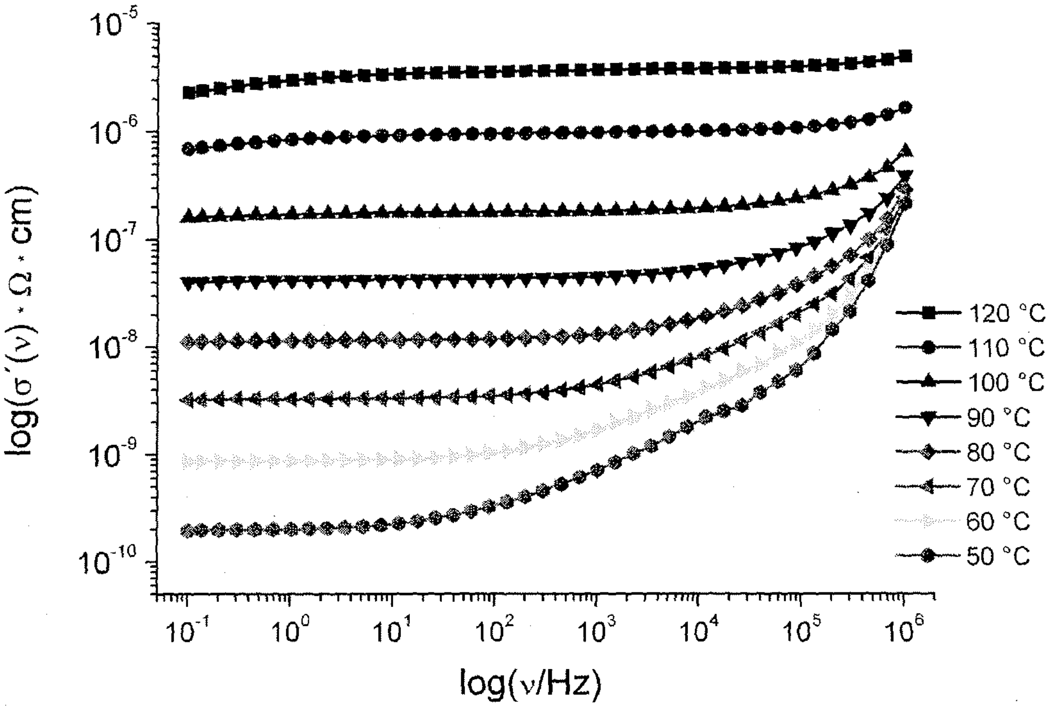
$10^{-5}$
$10^{-6}$
$10^{-7}$
$10^{-8}$
$10^{-9}$
$10^{-10}$
log(σ′(ν) · Ω · cm)
$10^{-1}$ $10^{0}$ $10^{1}$ $10^{2}$ $10^{3}$ $10^{4}$ $10^{5}$ $10^{6}$
log(ν/Hz)
120 °C
110 °C
100 °C
90 °C
80 °C
70 °C
60 °C
50 °C

Fig. 6

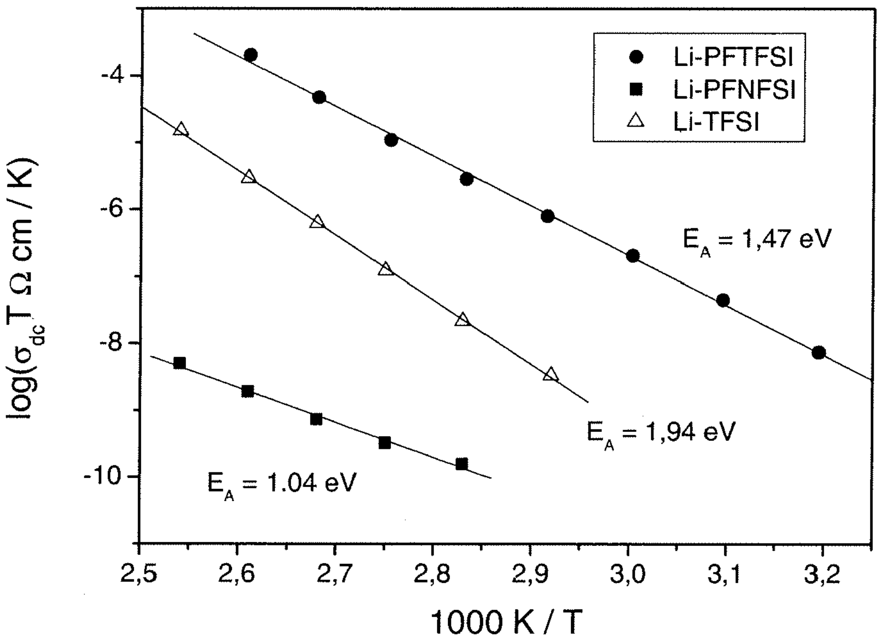
Li-PFTFSI
Li-PFNFSI
Li-TFSI
E_A = 1,47 eV
E_A = 1,94 eV
E_A = 1.04 eV
log(σ_dc T Ω cm / K)
1000 K / T

Fig. 7

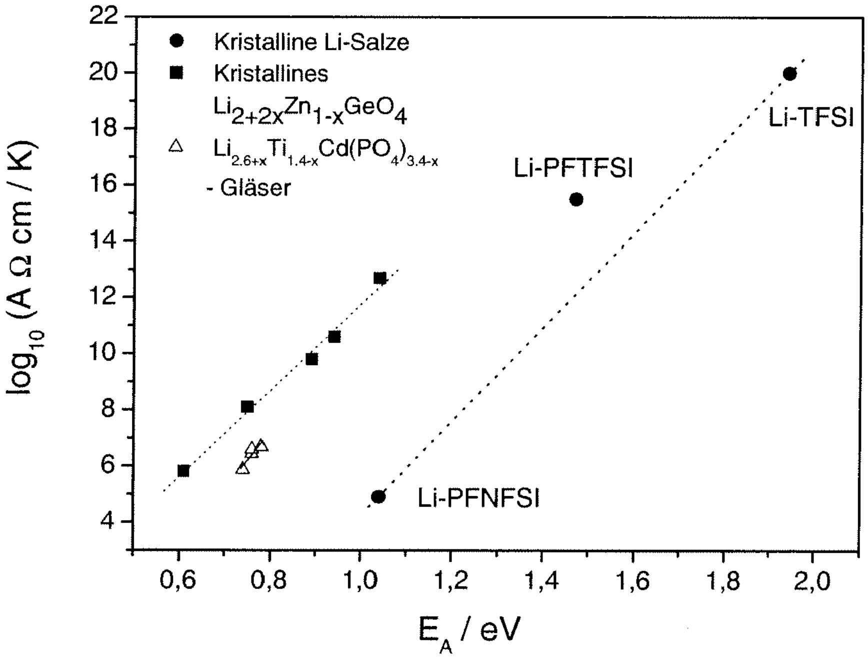
Kristalline Li-Salze
Kristallines
Li2+2xZn1-xGeO4
Li2.6+xTi1.4-xCd(PO4)3.4-x
- Gläser
Li-TFSI
Li-PFTFSI
Li-PFNFSI
log10 (A Ω cm / K)
E_A / eV

Fig. 8

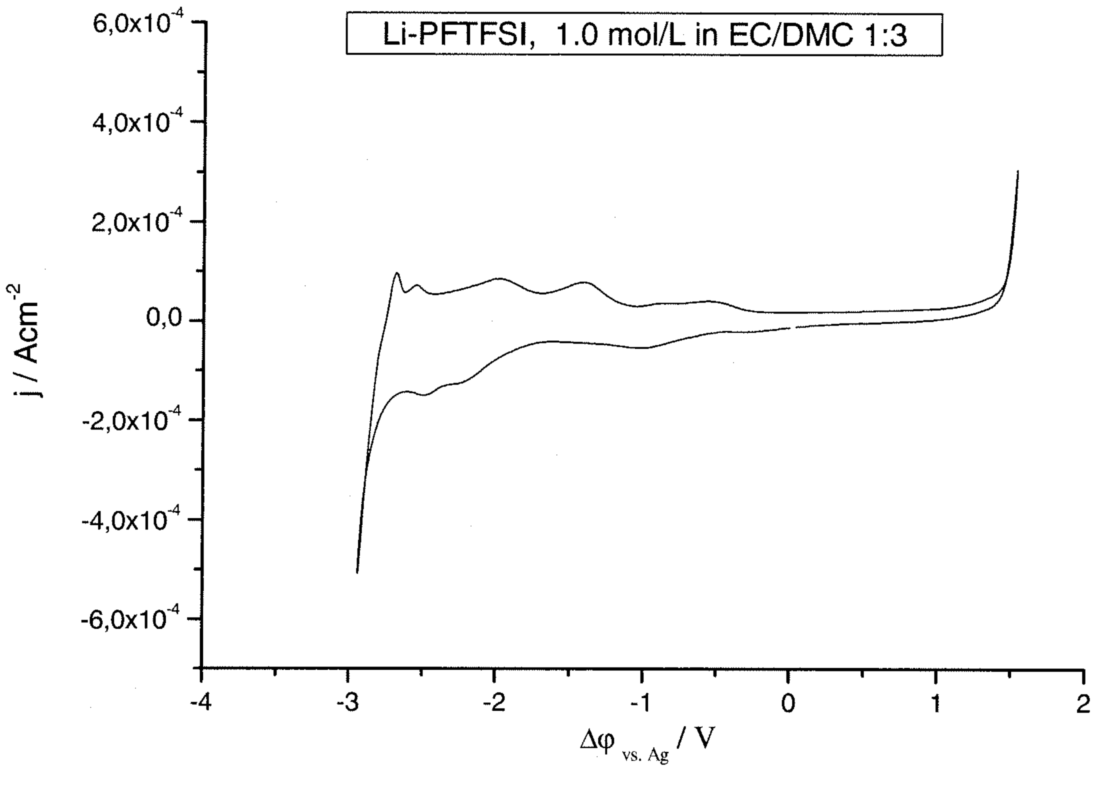
Li-PFTFSI, 1.0 mol/L in EC/DMC 1:3
6,0x10-4
4,0x10-4
2,0x10-4
0,0
-2,0x10-4
-4,0x10-4
-6,0x10-4
j / Acm-2
-4
-3
-2
-1
0
1
2
Δφ vs. Ag / V

Fig. 9

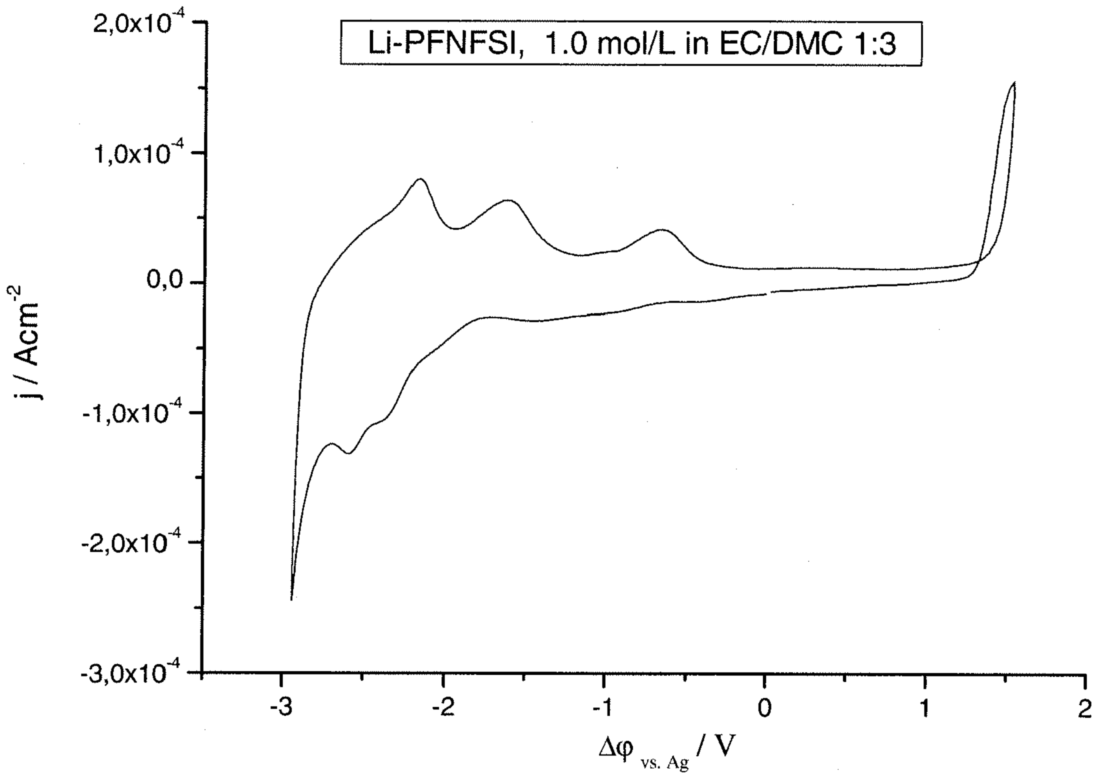
Li-PFNFSI, 1.0 mol/L in EC/DMC 1:3
2,0x10-4
1,0x10-4
0,0
-1,0x10-4
-2,0x10-4
-3,0x10-4
j / Acm-2
-3
-2
-1
0
1
2
Δφ vs. Ag / V

Fig. 10

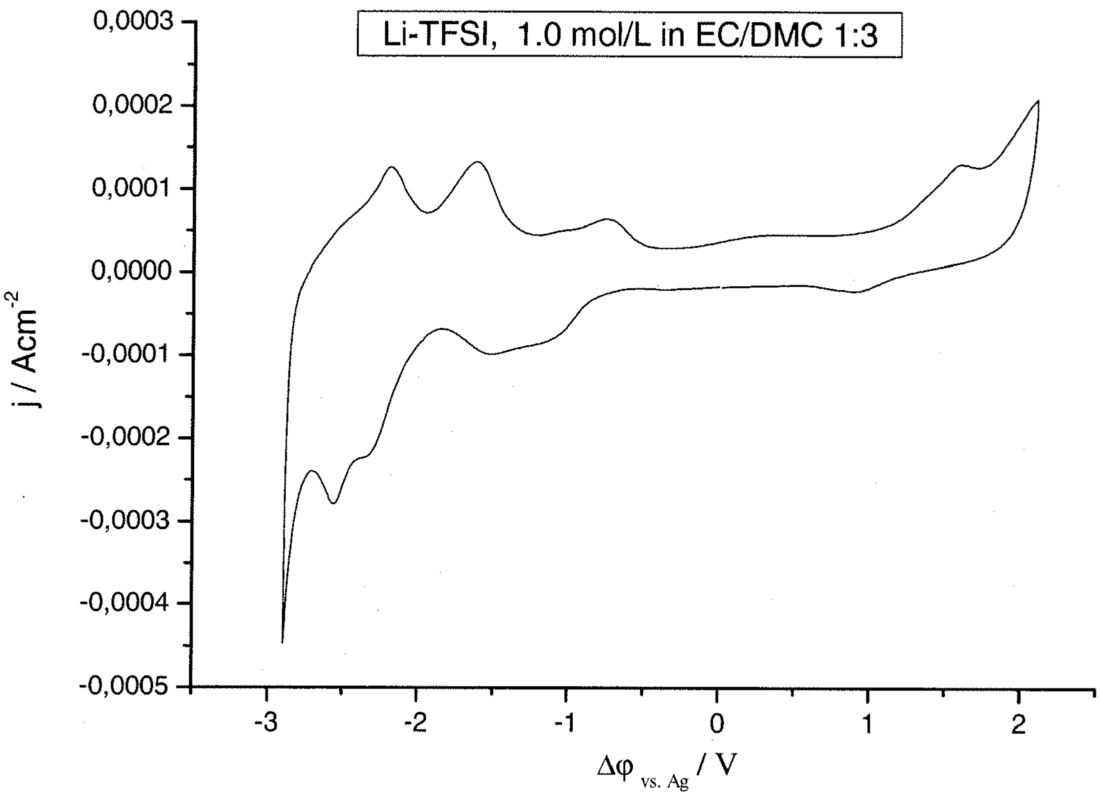
Li-TFSI, 1.0 mol/L in EC/DMC 1:3
0,0003
0,0002
0,0001
0,0000
-0,0001
-0,0002
-0,0003
-0,0004
-0,0005
j / Acm-2
-3
-2
-1
0
1
2
Δφ vs. Ag / V

Europäisches Patentamt
European Patent Office
Office européen des brevets

# EUROPÄISCHER RECHERCHENBERICHT

Nummer der Anmeldung
EP 09 17 3567

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | THOMAS LINDER AND JO RG SUNDERMEYER: "Three novel anions based on pentafluorophenyl amine combined with two new synthetic strategies for the synthesis of highly lipophilic ionic liquids" CHEMICAL COMMUNICATIONS - CHEMCOM, ROYAL SOCIETY OF CHEMISTRY, GB, 1. April 2009 (2009-04-01), Seiten 2914-2916, XP007912098 ISSN: 1359-7345 * Schemes 1,2; Seite 2915, linke Spalte * * Seite 2916, rechte Spalte * ----- | 1-2,9 | INV. C07C303/34 C07C303/40 C07C307/02 C07C311/08 C07C311/09 C07C311/10 C07C311/21 C07F1/02 H01M10/0525 H01M10/0568 |
| X,D | WO 2007/131498 A2 (UNIV MARBURG PHILIPPS [DE]; SUNDERMEYER JOERG [DE]; LINDER THOMAS [DE]) 22. November 2007 (2007-11-22) * Seite 10, Zeile 5 - Seite 12, Zeile 2; Ansprüche 1,18-19 * ----- | 1-9 | |
| A | UEMATSU N ET AL: "Synthesis of novel perfluorosulfonamide monomers and their application" JOURNAL OF FLUORINE CHEMISTRY, ELSEVIER, NL, Bd. 127, Nr. 8, 1. August 2006 (2006-08-01), Seiten 1087-1095, XP025184865 ISSN: 0022-1139 [gefunden am 2006-08-01] * Scheme 5 * ----- -/-- | 1-9 | RECHERCHIERTE SACHGEBIETE (IPC) C07C H01M C07F |

Der vorliegende Recherchenbericht wurde für alle Patentansprüche erstellt

| Recherchenort | Abschlußdatum der Recherche | Prüfer |
|---|---|---|
| München | 11. März 2010 | Steinreiber, J |

3

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 09 17 3567

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | "LÖSUNGSMITTEL" INTERNET CITATION 27. September 2009 (2009-09-27), XP007912113 Gefunden im Internet: URL:http://de.wikipedia.org/w/index.php?title=L%C3%B6sungsmittel&oldid=64976289> [gefunden am 2010-03-09] * Seiten 2,3,5 * ----- | 4-8 | |
| A,D | US 6 319 428 B1 (MICHOT CHRISTOPHE [FR] ET AL) 20. November 2001 (2001-11-20) * Anspruch 1; Beispiel 33 * ----- | 4-8 | |
| A | US 2007/010455 A1 (HEWAWASAM PIYASENA [US] ET AL HEWAWASAM PIYASENA [US] ET AL) 11. Januar 2007 (2007-01-11) * Absatz [0336] * ----- | 4-8 | |
| | | | RECHERCHIERTE SACHGEBIETE (IPC) |

Der vorliegende Recherchenbericht wurde für alle Patentansprüche erstellt

3

| Recherchenort | Abschlußdatum der Recherche | Prüfer |
|---|---|---|
| München | 11. März 2010 | Steinreiber, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 09 17 3567

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-03-2010

| Im Recherchenbericht angeführtes Patentdokument | Datum der Veröffentlichung | Mitglied(er) der Patentfamilie | Datum der Veröffentlichung |
|---|---|---|---|
| WO 2007131498 A2 | 22-11-2007 | DE 102006023649 A1 | 03-01-2008 |
| | | EP 2094667 A2 | 02-09-2009 |
| | | JP 2009537463 T | 29-10-2009 |
| | | US 2009298189 A1 | 03-12-2009 |
| US 6319428 B1 | 20-11-2001 | CA 2244979 A1 | 09-07-1998 |
| | | CA 2248242 A1 | 09-07-1998 |
| | | CA 2248244 A1 | 09-07-1998 |
| | | CA 2248246 A1 | 09-07-1998 |
| | | CA 2248303 A1 | 09-07-1998 |
| | | CA 2248304 A1 | 09-07-1998 |
| | | CA 2683826 A1 | 09-07-1998 |
| | | WO 9829358 A2 | 09-07-1998 |
| | | WO 9829399 A1 | 09-07-1998 |
| | | WO 9829389 A1 | 09-07-1998 |
| | | WO 9829396 A1 | 09-07-1998 |
| | | WO 9829877 A1 | 09-07-1998 |
| | | WO 9829388 A1 | 09-07-1998 |
| | | DE 69705301 D1 | 26-07-2001 |
| | | DE 69705301 T2 | 02-05-2002 |
| | | DE 69715361 D1 | 17-10-2002 |
| | | DE 69715361 T2 | 30-04-2003 |
| | | DE 69715799 D1 | 31-10-2002 |
| | | DE 69715799 T2 | 14-08-2003 |
| | | DE 69721748 D1 | 12-06-2003 |
| | | DE 69721748 T2 | 11-03-2004 |
| | | DE 69736994 T2 | 26-07-2007 |
| | | EP 1201650 A2 | 02-05-2002 |
| | | EP 1391952 A2 | 25-02-2004 |
| | | EP 0850920 A2 | 01-07-1998 |
| | | EP 0850933 A1 | 01-07-1998 |
| | | EP 0850921 A1 | 01-07-1998 |
| | | EP 0850932 A1 | 01-07-1998 |
| | | EP 0889863 A2 | 13-01-1999 |
| | | EP 0890176 A1 | 13-01-1999 |
| | | JP 4070244 B2 | 02-04-2008 |
| | | JP 2002514245 T | 14-05-2002 |
| | | JP 2000508676 T | 11-07-2000 |
| | | JP 4124487 B2 | 23-07-2008 |
| | | JP 2000508677 T | 11-07-2000 |
| | | JP 2000508346 T | 04-07-2000 |
| | | JP 4361137 B2 | 11-11-2009 |
| | | JP 2000508114 T | 27-06-2000 |
| | | JP 2000508678 T | 11-07-2000 |
| | | JP 2008007781 A | 17-01-2008 |
| | | JP 2009004374 A | 08-01-2009 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 09 17 3567

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-03-2010

| Im Recherchenbericht angeführtes Patentdokument | Datum der Veröffentlichung | Mitglied(er) der Patentfamilie | Datum der Veröffentlichung |
|---|---|---|---|
| US 6319428 B1 | | JP 2009149656 A | 09-07-2009 |
| | | JP 2009242401 A | 22-10-2009 |
| | | US 6333425 B1 | 25-12-2001 |
| | | US 6171522 B1 | 09-01-2001 |
| | | US 6120696 A | 19-09-2000 |
| | | US 6228942 B1 | 08-05-2001 |
| | | US 6395367 B1 | 28-05-2002 |
| | | US 6576159 B1 | 10-06-2003 |
| US 2007010455 A1 | 11-01-2007 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009003224 A1
- US 6319428 B1
- US 20070093678 A1
- WO 9526056 A
- WO 2007131498 A2


### In der Beschreibung aufgeführte Nicht-Patentliteratur


- **R Koppang.** Use of a Lithium Amide Suspension in Tetrahydrofuran for Preparation of Some Polyfluorophenyl- and Polyfluorodiphenylamines. *Acta Chem Scand,* 1971, vol. 25, 3067-3071
- **A Khvorost ; PL Shutov ; K Harms ; J Lorberth ; J Sundermeyer ; SS Karlov ; GS Zaitseva.** Lithium Bis(pentafluorophenyl)amides - Synthesis and Characterization of its Complexes with Diethyl Ether and THF. *Z. Anorg. Allg. Chem.,* 2004, vol. 630, 885-889
- **T Linder ; J Sundermeyer.** Three novel anions based on pentafluorophenyl amine combined with two new synthetic strategies for the synthesis of highly lipophilic ionic liquids. *Chem Commun,* 2009, 2914-2916